# EUROPÄISCHE PATENTSCHRIFT

(11) **EP 2 313 772 B1**
(45) Veröffentlichungstag und Bekanntmachung des Hinweises auf die Patenterteilung: **13.08.2014**
(21) Anmeldenummer: 09772115.3
(22) Anmeldetag: 26.06.2009
(51) Int. Cl.: C12N 5/0775

(54) **ISOLIERUNG UND/ODER IDENTIFIZIERUNG VON STAMMZELLEN MIT ADIPOZYTÄREM, CHONDROZYTÄREM UND PANKREATISCHEM DIFFERENZIERUNGSPOTENTIAL**
ISOLATION AND/OR IDENTIFICATION OF STEM CELLS HAVING ADIPOCYTIC, CHONDROCYTIC AND PANCREATIC DIFFERENTIATION POTENTIAL
ISOLEMENT ET/OU IDENTIFICATION DE CELLULES SOUCHES AYANT UN POTENTIEL DE DIFFÉRENTIATION ADIPOCYTAIRE, CHONDROCYTAIRE ET PANCRÉATIQUE

(30) Priorität: 30.06.2008 DE 102008032236
(43) Veröffentlichungstag der Anmeldung: 27.04.2011
(73) Patentinhaber: Eberhard-Karls-Universität Tübingen, 72076 Tübingen (DE)
(72) Erfinder: BUEHRING, Hans-Joerg, 72076 Tübingen (DE); TREML, Sabrina, 72108 Rottenburg a. N. (DE); LAMMERS, Reiner, 72070 Tübingen (DE)
(74) Vertreter: Witte, Weller & Partner Patentanwälte mbB
(86) Internationale Anmeldenummer: PCT/EP2009/004614
(87) Internationale Veröffentlichungsnummer: WO 2010/000415

(56) Entgegenhaltungen:
- EP-A- 1 901 063
- WO-A-2004/025293
- WO-A-2006/108229
- BUEHRING HANS-JOERG ET AL: "Novel markers for the prospective isolation of human MSC" ANNALS OF THE NEW YORK ACADEMY OF SCIENCES, NEW YORK ACADEMY OF SCIENCES, NEW YORK, NY, US, 16. September 2006 (2006-09-16), Seiten 262-271, XP009109140
- VOGEL WICHARD ET AL: "Heterogeneity among human bone marrow-derived mesenchymal stem cells and neural progenitor cells" HAEMATOLOGICA, FONDAZIONE FERRATA STORTI, ROME, IT, Bd. 88, Nr. 2, 1. Februar 2003 (2003-02-01), Seiten 126-133, XP002531561 ISSN: 0390-6078

## Beschreibung

Die vorliegende Erfindung betrifft die Isolierung und/oder Identifizierung von Stammzellen mit adipozytärem, chondrozytärem und pankreatischem Differenzierungspotential, sowie die Verwendung dieser Stammzellen.

Mesenchymale Stammzellen (MSC), auch mesenchymale stromale Zellen genannt, sind multipotente Zellen, die die Fähigkeit besitzen, unter geeigneten *in vitro-* und in *vivo*-Bedingungen in verschiedene mesenchymale Gewebe auszudifferenzieren. So können sie bspw. in Osteocyten, Chondrocyten, Adipozyten, Myocyten differenzieren, und Knochen-, Knorpel-, Fett-, und Muskelgewebe bilden. Darüber hinaus können sie sich aber auch in Astrozyten, Neurone, Endothelzellen, Hepatocyten, pankreas-ähnliche Zellen und Lungen-Epithelzellen differenzieren. Morphologisch sind sie an ihrem fibroblastoiden Phänotyp zu erkennen, und sind in verschiedenen adulten und embryonalen Geweben des Menschen zu finden, darunter im Gehirn, Knochenmark, Nabelschnurblut, in Blutgefäßen, im Skelettmuskel, der Haut, Leber, Zahnfleisch und der Plazenta.

MSC exprimieren eine Reihe von Oberflächenmarkern wie bspw. CD105 (Endoglin, SH2), CD73 (Ecto-S'-Nucleotidase, SH3, SH4), CD166 (ALCAM), CD29 (β1-Integrin), CD44 (H-CAM) und CD90 (Thy-1), die z.T. auch auf endothelialen, epithelialen Zellen sowie auf Muskelzellen gefunden werden. MSC lassen sich aber von hämatopoetischen Stammzellen abgrenzen, da MSC die für hämatopoetische Stammzellen spezifischen Marker CD45, CD34 und CD133 nicht exprimieren.

Mesenchymale Stammzellen haben die Eigenschaft, schnell und stabil auf Plastik- oder Glasoberflächen zu adhärieren, und koloniebildende Fibroblasten ("colony forming units" (CFU-F)) zu bilden. Letztere sind jedoch in Bezug auf ihre Proliferations- und Differenzierungsfähigkeiten heterogen.

Mesenchymale Stammzellen mit einem bestimmten Differenzierungspotential sind in der Medizin und Forschung von großem Interesse: Sie können insbesondere aus dem Knochenmark gewonnen werden, sogar aus jenem älterer Menschen, haben eine hohe Teilungsrate und können wie erwähnt in Gewebszellen mesenchymalen Ursprungs differenzieren. Sie könnten daher bspw. im Rahmen von Stammzelltherapien direkt der Behandlung degenerativer Erkrankungen von Organen wie Knochen, Knorpel, Sehnen, Muskel, Bindegewebe, Blutzellen, etc. dienen.

Zur Gewinnung bzw. Isolierung von mesenchymalen Stammzellen werden gegenwärtig unfraktionierte Knochenmarkszellen als Ausgangsmaterial verwendet, die auf Plastikschalen kultiviert, die MSC über deren Adhäsion an die Plastikoberfläche identifiziert, und die nicht-adhärierenden hämatopoetischen Zellen aus der Probe verworfen. Die auf diese Weise gewonnenen Zellen sind jedoch wenig definiert und differenzieren sich nicht nur in heterogene MSC Populationen, sondern auch in Osteoblasten, und/oder in Osteoblasten-Vorläuferzellen, in Fettzellen, Retikulumzellen, Makrophagen und Endothelzellen. Eine spezifische Behandlung degenerativer Erkrankungen eines Organs ist daher mit MSC ohne bestimmtes Differenzierungspotential schwierig bzw. auf Grund möglicher Nebenwirkungen unmöglich.

Die Isolierung von mesenchymalen Stammzellen mit einem ganz bestimmten Differenzierungspotential ist im Stand der Technik bisher nicht möglich oder bekannt. Eine solche Isolierung würde jedoch wie erwähnt den großen Vorteil bieten, dass derart identifizierte Stammzellen gezielt für die Therapie/Behandlung von erkranktem, degeneriertem oder beschädigtem Gewebe eingesetzt werden könnten, in welches sich die derart spezifisch isolierten Stammzellen differenzieren.

So könnten bspw. Knorpelschäden dadurch behandelt werden, dass spezifisch isolierte mesenchymale Stammzellen mit chondrogenem Differenzierungspotential entweder direkt *in situ* in das betroffene Gewebe gegeben werden, wo sie zu Chondrozyten ausdifferenzieren und dadurch das beschädigte Gewebe ersetzen (Stammzelltherapie). Andererseits kann aber auch eine Differenzierung in Chondrozyten *in vitro* von Interesse sein, wenn - bspw. für die Forschung/Diagnostik/Medizin - ausdifferenzierte Chondrozyten gewonnen werden sollen.

Vor diesem Hintergrund besteht ein großes Interesse an mesenchymalen Stammzellen mit einem bestimmten Differenzierungspotential, insbesondere um diese in entsprechenden Verwendungen einzusetzen.

Aufgabe der vorliegenden Erfindung ist daher, neue Wege bereitzustellen, mit denen mesenchymale Stammzellen mit einem bestimmten Differenzierungspotential isoliert werden können.

Erfindungsgemäß wird diese Aufgabe gelöst durch die Verwendung eines Antikörpers, der an das Antigen TNAP (Tissue non-specific Alkaline Phosphatase; Gewebeunspezifische Alkalische Phosphatase) bindet; insbesondere wird die Aufgabe gelöst durch die Verwendung eines Antikörpers, oder funktionellen Fragmenten des Antikörpers, der an das Antigen TNAP bindet, in Kombination mit einem Antikörper, oder funktionellen Fragmenten des Antikörpers, der an CD56 bindet, zur Isolierung von Stammzellen mit adipozytärem, chondrozytärem und pankreatischem Differenzierungspotential.

Die Aufgabe wird ferner gelöst durch ein Verfahren zur Isolierung und/oder Identifizierung von mesenchymalen Stammzellen mit chondrozytärem, adipozytärem oder pankreatischem Differenzierungspotential, bei welchen die gegen TNAP und gegen CD56 bindenden Antikörper eingesetzt werden.

Ferner betrifft die Offenbarung die auf diese Weise isolierten Stammzellen und deren Verwendung, insbesondere in der Therapie.

Die der Erfindung zugrunde liegende Aufgabe wird auf diese Weise vollkommen gelöst. Die Erfinder der vorliegenden Anmeldung konnten in eigenen Versuchen zeigen, dass es unter Verwendung der genannten Antikörper möglich ist, spezifisch mesenchymale Stammzellen zu isolieren, die anschließend in Chondrozyten, Adipozyten oder Pankreas-ähnliche Zellen differenzierten.

Die Erfinder konnten insbesondere nachweisen, dass die genannten Stammzellen spezifisch über das Antigen TNAP isoliert und/oder identifiziert werden können. Diese Isoform des Ecto-Enzyms "alkalische Phosphatase" wird aufgrund ihres Vorkommens auch als "Leber/Knochen/Nieren alkalische Phosphatase" bezeichnet. Die drei weiteren Isoformen sind Darm-, Plazenta- und Plazenta-ähnliche alkalische Phosphatase. TNAP unterscheidet sich von den anderen Isoformen durch posttranslationale Modifikationen und durch die Genlokalisation.

Damit wird zum ersten Mal ein Werkzeug bereitgestellt, mit welchem mesenchymale Stammzellen gewonnen werden können, die spezifisch ausdifferenzieren. Dies war bisher im Stand der Technik nicht möglich.

Aufgrund der neuen Verwendung und des neuen Verfahrens können also mesenchymale Stammzellen bereitgestellt werden, die bspw. wiederum vorteilhafterweise in der Therapie und Prophylaxe oder aber in der Diagnostik und Forschung eingesetzt werden können. So können die derart isolierten Stammzellen insbesondere zur Behandlung von Krankheiten, die sich durch ein degeneriertes, verletztes oder beschädigtes Gewebe auszeichnen, eingesetzt werden, bspw. im Rahmen einer Stammzelltherapie: Die mittels des erfindungsgemäßen Verfahrens isolierten Stammzellen werden hierfür in das betroffene Gewebe transplantiert (bspw. auch im Zusammenhang mit bestimmten Inplantaten), und differenzieren sich dort in das entsprechende Gewebe. Dadurch wird das degenerierte Gewebe regeneriert und wieder funktionsfähig.

In einer Weiterbildung der erfindungsgemäßen Verwendung ist dabei bevorzugt wenn der anti-TNAP-Antikörper ausgewählt ist aus der Gruppe:
- der Antikörper W8B2, der von der bei der Deutschen Sammlung von Mikroorganismen und Zellkulturen hinterlegten Zelllinie mit der Nr. ACC 2567 produziert wird,
- funktionelle Fragmente des Antikörpers W8B2, die an das Antigen TNAP binden, der von der bei der Deutschen Sammlung von Mikroorganismen und Zellkulturen hinterlegten Zelllinie mit der Nr. ACC 2567 produziert wird, und
- ein Antikörper, der an das gleiche Epitop bindet wie der Antikörper W8B2, der von der bei der Deutschen Sammlung von Mikroorganismen und Zellkulturen hinterlegten Zelllinie mit der Nr. ACC 2567 produziert wird.

Der TNAP-spezifische Antikörper W8B2 hat sich in anderen Studien als Marker für mesenchymale Stammzellen erwiesen; so konnten in einer anderen Erfindung des Anmelders (DE 10 2006 043 625) die Erfinder zeigen, dass es mittels des Antikörpers W8B2 möglich ist, mesenchymale Stammzellen aus Primärgewebe zu isolieren. Allerdings war bisher nicht bekannt, dass mit einer Kombination von Antikörpern, die gegen TNAP und CD56 gerichtet sind, mesenchymale Stammzellen mit ganz bestimmten Differenzierungspotentialen gewonnen werden können. Die den Antikörper W8B2 produzierenden Zellen wurden bei der Deutschen Sammlung für Mikroorganismen und Zellkulturen (DSMZ) gemäß dem Budapester Vertrag hinterlegt. Der Antikörper wurde durch eine Immunisierung von Balb/c Mäusen mit der Retinoblastom-Zelllinie WERI-RB-1 gewonnnen. Ursprünglich war das durch den Antikörper W8B2 erkannte Antigen als mesenchymales Stammzellen Antigen-1 bezeichnet worden; zwischenzeitlich wurde aber das Antigen, das durch den Antikörper W8B2 erkannt wird, als TNAP identifiziert, weshalb vorliegend die korrekte und offizielle Bezeichnung des Antigens gewählt wurde.

In einer bevorzugten Ausführungsform ist der an CD56 bindende Antikörper ausgewählt aus der Gruppe:
- der Antikörper 39D5, der von der bei der Deutschen Sammlung von Mikroorganismen und Zellkulturen hinterlegten Zelllinie mit der Nr, ACC 2930 produziert wird,
- funktionelle Fragmente des Antikörpers 39D5, die an CD56 binden, der von der bei der Deutschen Sammlung von Mikroorganismen und Zellkulturen hinterlegten Zelllinie Nr. ACC 2930 produziert wird,
- ein Antikörper, der an das gleiche Antigen bindet wie der Antikörper 39D5, der von der bei der Deutschen Sammlung von Mikroorganismen und Zellkulturen hinterlegten Zelllinie Nr. ACC 2930 produziert wird,
   und
- ein Antikörper, der an das gleiche Epitop bindet wie der Antikörper 39D5, der von der bei der Deutschen Sammlung von Mikroorganismen und Zellkulturen hinterlegten Zellllnie Nr. ACC 2930 produziert wird.

Der Antikörper 39D5, der ebenfalls bei der Deutschen Sammlung von Mikroorganismen und Zellkulturen gemäß dem Budapester Vertrag unter der Nr. ACC 2930 hinterlegt worden ist, wurde durch eine Immunisierung von Balb/c Mäusen mit der hämatopoetischen Zelllinie KG-1a gewonnen.

In einer Ausführungsform der erfindungsgemäßen Verwendung ist bevorzugt, wenn zu der Kombination aus einem anti-TNAP und einem gegen CD56 gerichteten Antikörper zusätzlich noch ein gegen CD271 gerichteter Antikörper, oder funktionelle Fragmente des Antikörpers, eingesetzt wird.

Anti-CD271-Antiköprer in konjugierter Form sind im Stand der Technik bekannt, und bspw. erhältlich von Miltenyi Biotech, Bergisch Gladbach, Deutschland.

Das CD271 Antigen, das auch als LNGFR ("low-affinity nerve growth factor receptor") oder p75 NTR (Neurotrophin Rezeptor) bekannt ist, gehört zur den Neurotrophin-Rezeptoren mit niedriger Affinität und zu der Superfamilie der Tumornekrosefaktor-Rezeptoren. Ursprünglich wurde CD271 (LNGFR) als Marker von Zellen des Nervensystems beschrieben, da CD271 (LNGFR) im zentralen und peripheren Nervensystem auf autonomen und sensorischen Neuronen gefunden wird. Daneben wird CD271 auch auf Oligodendrozyten, Astrozyten und Schwann'sche Zellen exprimiert. Ferner findet sich CD271 (LNGFR) aber auch auf bestimmten MSC.

Vorliegend sollen die Begriffe "funktionale Fragmente" oder "funktionelle Fragmente", wie sie in der Anmeldung verwendet werden, Substanzen bedeuten, die Teile/Abschnitte der offenbarten Antikörper darstellen und die immer noch die funktionellen Eigenschaften, insbesondere die Zell-Bindungseigenschaften der Antikörper zeigen und besitzen, von denen sie abgeleitet sind. Dabei können diese Fragmente entweder als solche oder aber in Kombination mit anderen Fragmenten eingesetzt werden; im Rahmen der vorliegenden Erfindung sind zu letzteren bspw. auch modifizierte W8B2 oder 39D5 Antikörper zu verstehen, die für entsprechende Einsätze und Verwendungen beim Menschen angepasst, bspw. humanisiert wurden.

Die für die Zwecke der vorliegenden Erfindung geeigneten Antikörper sind vorzugsweise monoklonal, wobei weitere, gegen TNAP oder CD56 gerichtete Antikörper unter Einsatz der Antikörper W8B2 und 39D5 gewonnen werden können. Eine Anleitung zur Gewinnung von monoklonalen Antikörpern veröffentlichten Köhler und Milstein ("Continuous cultures of fused cells secreting antibody of predefined specificity", Nature, (1975), 256:495-497).

Vorliegend sind aber auch Fragmente solcher Antikörper, wie bspw. Fab, F(ab)'₂ oder scFv Fragmente, und andere Fragmente wie CDR ("complementarity-determining region"), hypervariable Region) als Antikörper im Sinne und Rahmen der vorliegenden Erfindung gemeint, so lange wie sie ihre Funktionalität, d.h. die spezifischen Bindungseigenschaften wie die "ganzen" Antikörper, von denen sie abgeleitet sind, besitzen. Solche Antikörper-Fragmente können bspw. auch rekombinant unter Verwendung im Stand der Technik bekannter Verfahren hergestellt werden.

Daher versteht sich auch, dass die Antikörper W8B2 und 39D5 andererseits auch entsprechend humanisiert werden können, und im Rahmen der hier offenbarten Erfindung für die erfindungsgemäßen Verwendungen und/oder Verfahren, insbesondere auch für eine Stammzelltherapie, eingesetzt werden können.

Humanisierte Antikörper können bspw. chimäre Antikörper sein, bei welchen die konstanten Regionen der tierischen Antikörper (bspw. von Maus- oder Kaninchen-Antikörpern) durch die entsprechenden Regionen menschlicher Antikörper, bspw. den Fc-Fragmenten, ersetzt wurden (Sharon et al., Nature, (1984), 309:364-367). Alternativ kann auch die CDR der tierischen Antikörper mit menschlichen Antikörpern verbunden werden, dieser Prozess wird Antikörper "Reshaping" genannt. In einem weiteren anderen Verfahren werden in transgenen Tieren menschliche Antikörper produziert.

Ferner betrifft die Offenbarung eine Verwendung der Antikörper, bspw. in humanisierter Form, oder funktionelle Fragmente davon, bei der diese auf entsprechende implantierbare medizinische Vorrichtungen auf- oder eingebracht werden, und zusammen mit der Vorrichtung in den zu behandelnden Patienten an die zu behandelnden Stellen/Gewebedefekten implantiert werden. An den zu behandelnden Stellen werden dann über die Antikörper mesenchymale Stammzellen rekrutiert, die sich an dem Implantat festsetzen, ausdifferenzieren und so neues Gewebe bilden. Geeignete medizinische Vorrichtungen sind hierbei irgendwelche biokompatiblen Implantate, Endoprothesen, bspw. Stents, etc., jeglicher Art, die entweder dauerhaft oder temporär in den Patienten eingebracht werden. Die Vorrichtungen können optional auch aus vollständig oder teilweise resorbierbaren Materialien bestehen und neben den Antikörpern weitere therapeutische Aktive Substanzen aufweisen, die üblicherweise bei in einen Körper einzubringenden Implantaten/Transplantaten eingesetzt werden.

Wie bereits weiter oben erwähnt, betrifft die vorliegende Erfindung auch ein Verfahren zur Isolierung und/oder Identifizierung von mesenchymalen Stammzellen mit adipozytärem Differenzierungspotential, wobei es die folgenden Schritte aufweist:
a) in-Kontakt-Bringen einer Probe, die mesenchymale Stammzellen enthält, mit einem Antikörper oder mit funktionellen Fragmenten des Antikörpers, der an das Antigen TNAP bindet,
b) in-Kontakt-Bringen der Probe aus Schritt a) mit einem Antikörper oder mit funktionellen Fragmenten des Antikörpers, der an CD56 bindet, und
c) Isolieren und/oder Identifizieren von Zellen, an welche der Antikörper oder funktionelle Fragmente des Antikörpers, der an das Antigen TNAP bindet, nicht aber der Antikörper oder funktionelle Fragmente des Antikörpers, der an CD56 bindet, gebunden hat.

Mit dieser Ausführungsform des erfindungsgemäßen Verfahrens können also Stammzellen gewonnen werden, die gezielt in Adipozyten differenzieren. Diese wiederum können für ganz bestimmte Zwecke, sei es in der Forschung oder Medizin, eingesetzt werden.

Ferner betrifft die vorliegende Erfindung ein Verfahren zur Isolierung und/oder Identifizierung von mesenchymalen Stammzellen mit chondrozytärem oder pankreatischem Differenzierungspotential, wobei das Verfahren die folgenden Schritte aufweist:
a) in-Kontakt-Bringen einer Probe, die mesenchymale Stammzellen enthält, mit einem Antikörper oder mit funktionellen Fragmenten des Antikörpers, der an das Antigen TNAP bindet,
b) in-Kontakt-Bringen der Probe aus Schritt a) mit einem Antikörper oder mit funktionellen Fragmenten des Antikörpers, der an CD56 bindet, und
c) Isolieren und/oder Identifizieren von Zellen, an welche sowohl der Antikörper oder funktionelle Fragmente des Antikörpers, der an das Antigen TNAP bindet, als auch der Antikörper oder funktionelle Fragmente des Antikörpers, der an CD56 bindet, gebunden haben.

Mit dieser Ausführungsform des erfindungsgemäßen Verfahrens können Stammzellen identifiziert und/oder isoliert werden, die ein chondrogenes oder pankreatisches Differenzierungspotential besitzen. Die derart gewonnen Stammzellen können dann entweder direkt im Rahmen einer Stammzelltherapie (autologe oder allogene Therapie) eingesetzt werden, wo sie *in situ* in Chondrozyten oder Pankreas-ähnliche Zellen differenzieren, und somit degeneriertes oder beschädigtes Knorpelgewebe oder Pankreasgewebe regenerieren können.

Andererseits können die mit dem erfindungsgemäßen Verfahren gewonnenen mesenchymalen Stammzellen mit chondrogenem/pankreatischem Differenzierungs-potential auch zunächst *in vitro* zu Chondrozyten/Pankreas-ähnliche Zellen differenziert werden, und anschließend zur Behandlung von erkranktem oder degeneriertem Gewebe eingesetzt werden.

Insbesondere in den letzten Jahren hat sich die autologe Chondrozyten Transplantation zu einem bevorzugten Eingriff zur Behandlung von (Gelenk-)Knorpeldefekten von Bandscheibe und Knie entwickelt, bei welchen der hyaline Knorpel wiederhergestellt werden sollte. Hierzu werden dem Patienten durch eine Arthroskopie aus einem nicht beschädigten Gelenkanteil Proben entnommen, und die darin enthaltenen Knorpelzellen im Labor auf speziellen Matrices gezüchtet. Das dadurch entstehende Gewebe, also der neue Knorpel, wird dann durch eine gewebeschonende zweite Operation in das erkrankte/degenerierte Gelenk transplantiert.

Mit dem erfindungsgemäßen Verfahren ist es nun zum ersten Mal möglich, gezielt Stammzellen mit bspw. chondrogenem Differenzierungspotential aus dem Gewebe eines Patienten zu isolieren und eine schnelle, effiziente und gezielte Anzüchtung von Chondrocyten-Gewebe für die nachfolgende Transplantation in den Proben-Spender (autologe Transplantation) oder einen anderen Empfänger (allogene Transplantation) zu erreichen.

Bei den erfindungsgemäßen Verfahren ist dabei bevorzugt, wenn der in Schritt a) eingesetzte anti-TNAP-Antikörper ausgewählt ist aus der Gruppe:
- der Antikörper W8B2, der von der bei der Deutschen Sammlung von Mikroorganismen und Zellkulturen hinterlegten Hybridomzelllinie mit der Nr. ACC 2567 produziert wird,
- funktionelle Fragmente des Antikörpers W8B2, die an das Anitgen TNAP binden, und
- Antikörper, die an das gleiche Epitop binden wie der Antikörper W8B2, der von der bei der Deutschen Sammlung von Mikroorganismen und Zellkulturen hinterlegten Hybridomzelllinie mit der Nr. ACC 2567 produziert wird.

Ferner ist in einer Weiterbildung der erfindungsgemäßen Verfahren bevorzugt, wenn der in Schritt b) eingesetzte an CD56 bindende Antikörper ausgewählt ist aus der Gruppe:
- der Antikörper 39D5, der von der bei der Deutschen Sammlung von Mikroorganismen und Zellkulturen hinterlegten Hybridomzelllinie Nr. ACC 2930 produziert wird,
- funktionelle Fragmente des Antikörpers 39D5, die an CD56 binden, und
- Antikörper, die an das gleiche Epitop binden wie der Antikörper 39D5, der von der bei der Deutschen Sammlung von Mikroorganismen und Zellkulturen hinterlegten Hybridomzelllinie Nr. ACC 2930 produziert wird.

In einer weiteren Ausführungsform des erfindungsgemäßen Verfahrens ist dabei bevorzugt, wenn es den weiteren Schritt c') aufweist:
c') in-Kontakt-Bringen der Probe aus Schritt b) mit einem Antikörper oder mit funktionellen Fragmenten des Antikörpers, der an CD271 bindet.

Dabei kann bei den erfindungsgemäßen Verfahren gemäß der Erfindung vorgesehen sein, wenn die Schritte a),b) und c') gleichzeitig, aufeinanderfolgend, in gemischter oder in umgekehrter Reihenfolge durchgeführt werden.

Die Erfinder haben in eigenen Versuchen festgestellt, dass unter Verwendung der Antikörper W8B2 und 39D5 in einem Verfahren zur Isolierung/Identifizierung von mesenchymalen Stammzellen eine gezielte Anreicherung von mesenchymalen Stammzellen mit chondrogenem/adipozytärem oder pankreatischem Differenzierungspotential gewonnen werden konnte.

Die vorliegende Erfindung betrifft ferner TNAP⁺ CD56⁺ Stammzellen, die mit den erfindungsgemäßen Verfahren zur Isolierung und/oder Identifizierung von mesenchymalen Stammzellen mit chondrozytärem oder pankreatischem Differenzierungspotential isoliert und/oder identifiziert wurden zur Anwendung für die Therapie, Diagnostik oder Forschung.

Die Offenbarung betrifft eine Ausführungsform, bei der die mit den erfindungsgemäßen Verfahren gewonnenen TNAP⁺ CD56⁺ Stammzellen zur definierten Generierung von Chondrozyten, Adipozyten und Pankreas-ähnlichen Zellen eingesetzt werden, und zwar *in vivo* oder *in vitro.*

Ferner ist in einer weiteren Ausführungsform bevorzugt, wenn die mit den erfindungsgemäßen Verfahren isolierten und/oder identifizierten TNAP⁺ CD56⁺ Stammzellen, die und zu Chondrozyten, Adipozyten und Pankreas-ähnlichen Zellen differenziert wurden, zur Therapie und/oder Prophylaxe von degeneriertem oder anfälligem Gewebe eingesetzt werden können.

Insbesondere ist bevorzugt, wenn die mit den erfindungsgemäßen Verfahren gewonnenen TNAP⁺ CD56⁺ Stammzellen zur Anwendung für die Therapie und/oder Prophylaxe von Knorpel- und/oder Knochenschäden, -degenerationen oder -erkrankungen, insbesondere der Knie und Bandscheiben, oder für rheumatoide Arthritis eingesetzt werden. Rheumatoide Arthritis stellt eine Autoimmunerkrankung dar, und auch bei dieser Krankheit kann die Verwendung von Stammzellen zum Gewebeersatz (also zum so genannten "tissue repair") zum Einsatz kommen.

Die Erfindung betrifft ferner eine pharmazeutische Zusammensetzung sowie ein Kit, die/das eine Kombination des Antikörpers W8B2, der von der bei der Deutschen Sammlung von Mikroorganismen und Zellkulturen hinterlegten Hybridomzelllinie mit der Nr. ACC 2567 produziert wird, oder Fragmenten davon, und des Antikörpers 39D5 aufweist, der von der bei der Deutschen Sammlung von Mikroorganismen und Zellkulturen hinterlegten Hybridomzelllinie Nr. ACC 2930 produziert wird, oder Fragmenten davon.

Ferner betrifft die Erfindung eine pharmazeutische Zusammensetzung, enthaltend TNAP⁺ CD56⁺ Stammzellen, die gemäß den erfindungsgemäßen Verfahren isoliert und/oder identifiziert wurden, sowie zumindest einen pharmazeutisch akzeptierbaren Träger- und/oder Hilfsstoff, und ggf. therapeutisch wirksame Substanzen.

Unter "pharmazeutisch akzeptierbaren Träger- oder Hilfsstoffen" wird vorliegend jede in der Pharmazie im Zusammenhang mit an einen Patienten zu verabreichende Substanz/Zusammensetzung verstanden, die die Wirksamkeit der Zellen/Antikörper nicht nachteilig beeinflusst, und/oder die pharmakologisch die Anwendung der pharmazeutischen Zusammensetzung unterstützen oder erleichtern kann.

Unter "therapeutisch wirksamer Substanz" wird vorliegend jede Substanz verstanden, die für die Zwecke einer Behandlung oder Verbesserung eines Krankheitsbildes eines Patienten eingesetzt wird.

Die pharmazeutischen Zusammensetzungen können systemisch verabreicht werden, d.h. bspw. oral, subkutan, intravenös, rektal, parenteral, intramuskulär, intraperitoneal, transdermal, oder topisch, wobei die Verabreichungsart von der Art der Erkrankung, dem Krankheitsbild, sowie dem Zustand der Patienten abhängen wird. Ebenso kann die Verabreichung wiederholt oder einmalig stattfinden, wobei die Verabreichung im ersteren Fall einmal oder mehrmals am Tag, und/oder über einen längeren Zeitraum hinweg erfolgen kann.

Zusätzlich zu den aktiven Substanzen kann die pharmazeutische Zusammensetzung auch noch Puffer, Verdünnungsmittel und/oder Additive enthalten. Geeignete Puffer schließen bspw. Tris-HCl, Glycin und Phosphat mit ein, und geeignete Verdünnungsmittel bspw. wässrige NaCl-Lösungen, Lactose oder Mannitol. Geeignete Additive schließen bspw. Detergentien, Lösungsmittel, Antioxidantien und Konservierungsstoffe mit ein. Eine Übersicht über solche zusätzlichen Inhaltsstoffe findet sich bspw. in A. Kibbe.: "Handbook of Pharmaceutical Excipients", 3rd Ed., 2000, American Pharmaceutical Association and Pharmaceutical Press.

Es versteht sich, dass die oben genannten und nachstehend noch zu erläuternden Merkmale nicht nur in der jeweils angegebenen Kombination, sondern auch in anderen Kombinationen oder in Alleinstellung verwendet werden können, ohne den Rahmen der vorliegenden Erfindung zu verlassen.

Die Erfindung wird in der nachstehenden Beschreibung und den beigefügten Figuren näher erläutert.

Es zeigen:

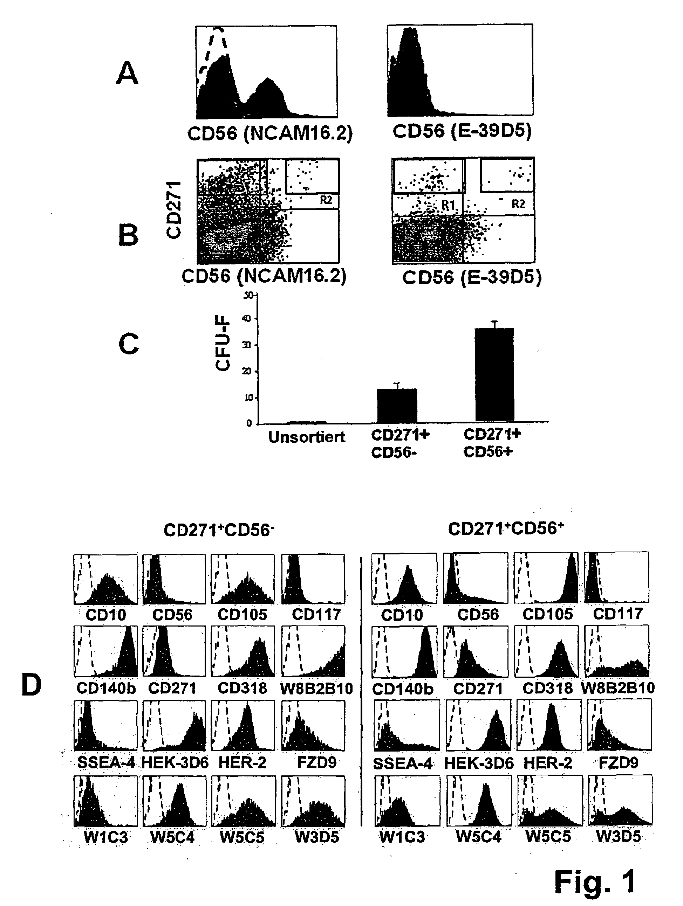

- Fig. 1: Charakterisierung der CD56⁺ Knochenmarkszellen.
(A) Das CD56-Epitop NCAM16.2, jedoch nicht das von 39D5, ist auf peripheren Blut-NK-Zellen exprimiert. (B) die CD56-Epitope NCAM16.2 und 39D5 sind auf einem seltenen CD271⁺ Knochenmarks-Subset exprimiert. (C) CD271⁺CD56⁺ und CD271⁺CD56⁻ Knochenmarkszellen sind klonogen. CFU-F, abgeleitet aus 500 FACS-sortierten Zellen, wurden gefärbt und wie beschrieben ausgewertet. Die Daten stellen den Mittelwert der CFU-F-Anzahlen dreier unterschiedlicher Versuche dar (* = p <0,01). (D) Expression selektierter Marker auf kultivierten CD271⁺CD56⁻ und CD271⁺CD56⁺ MSC.

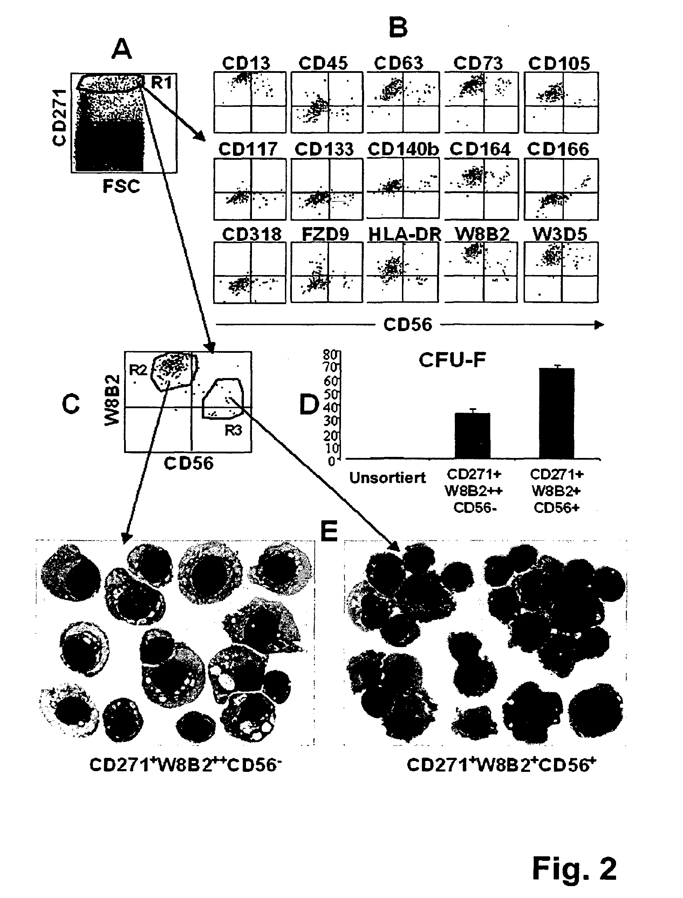

- Fig.2: Phänotyp und Morphologie von CD271^{bright}TNAP⁺CD56⁻ und CD271^{bright}TNAP⁺CD56⁺, abgeleitet aus dem Knochenmark.
Dreifach gefärbte Knochenmarkzellen wurden auf das CD271⁺-Subset eingeschränkt und hinsichtlich der Koexpression von CD56 und ausgewählten Markern analysiert. (A) Darstellung von FSC gegen CD271-APC. (B) Darstellung von CD56 gegen die dargestellten Marker auf CD271^{bright}-eingeschränkte Zellen. (C) Darstellung von CD56 gegen TNAP (W8B2) auf CD271^{bright}-Zellen. Die Sortierfenster werden mit R2 und R3 bezeichnet. (D) CFU-F Anzahlen, abgeleitet aus 1.000 FACS-sortierten BM TNAP⁺CD56⁻ und TNAP⁺CD56⁺ Zellen oder 100.000 unfraktionierten Knochenmarkzellen. Die resultierenden CFU-F wurden gefärbt und 12 Tage nach Kultur ausgewertet, und normalisiert auf 1.000 ausplattierte Zellen (p <0,01). (E) Morphologie von TNAP⁺CDS6⁻ und TNAP⁺CD56⁺ Knochenmarkellen. Die Subsets wurden sortiert, zytozentrifugiert, und mit May-Grünwald-Giemsa-Lösung gefärbt; anschließend wurden sie mit einem Zeiss Axiovert 200 Mikroskop ausgewertet.

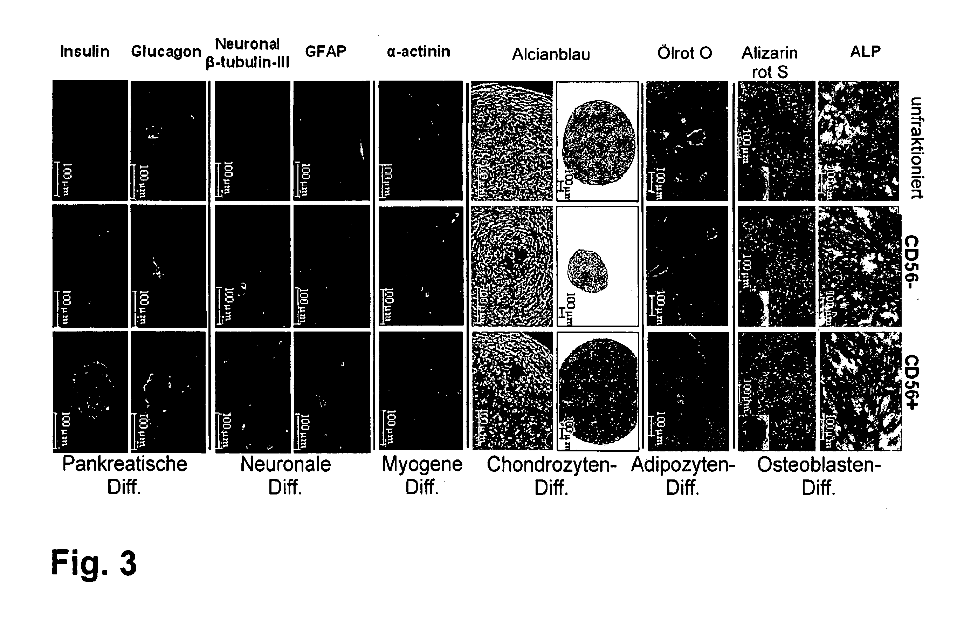

- Fig. 3: Differenzierungspotential von MSC, die aus sortierten TNAP⁺CD56⁻ und TNAP⁺CD56⁺ Knochenmarkszellen abgeleitet sind.
Zellen in Dreifachfärbung mit CD271, TNAP (W8B2) und CD56 wurden sortiert und wie beschrieben kultiviert. Expandierte MSC wurden zur osteogenen, adipogenen, chondrogenen, myogenen, neurogenen und pankreatischen Differenzierung induziert und wie beschrieben gefärbt. Die resultierenden Zellen wurden mit einem Zeiss Axiovert 200 Mikroskop fotografiert.

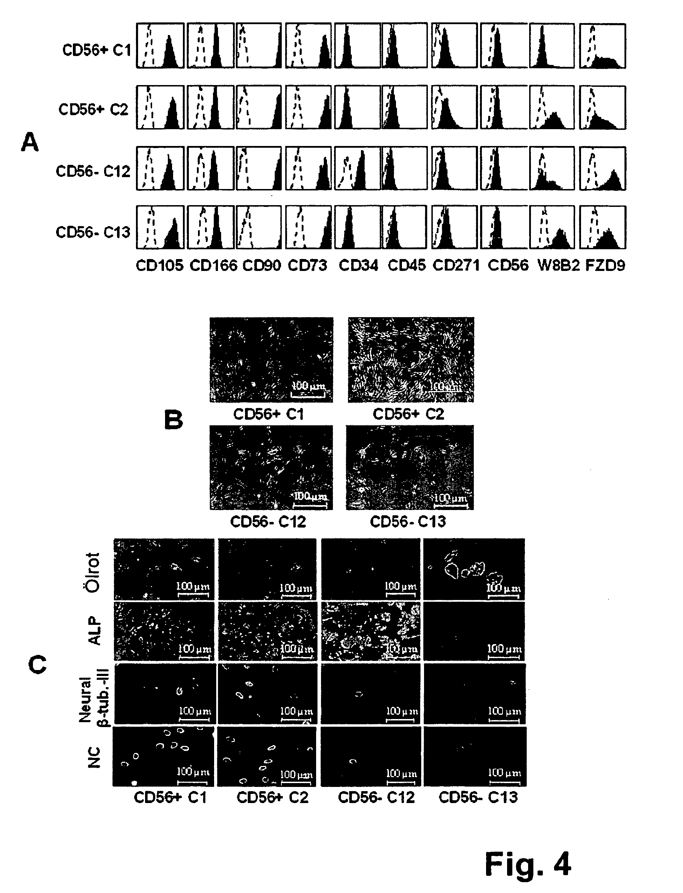

- Fig. 4: Phänotyp und Differenzierungsfähigkeit von MSC, abgeleitet aus Einzelzellen.
(A) Knochenmarkszellen, dreifach gefärbt mit CD56-FITC, TNAP-PE und CD271-APC wurden eingegrenzt und wie in Fig. 2C beschrieben sortiert. Die Einzelzellen wurden in 96-Well-Platten sortiert und 12 Tage lang kultiviert. Die entstehenden MSC-Kolonien wurden in T-25 Flaschen übertragen und weitere 12 Tage kultiviert; anschließend wurden sie mit den jeweiligen Antikörpern gefärbt. (B) Morphologie der TNAP⁺CD56⁺ abgeleiteten MSC-Klone C1 und C2, und der TNAP⁺CD56⁻ -abgeleiteten Klone C12 und C13. (C) Osteogenes, adipogenes und neuronales Differenzierungspotential der MSC, abgeleitet aus einzelnen TNAP⁺CD56⁻ und TNAP⁺CD56⁺ Knochenmarkszellen.

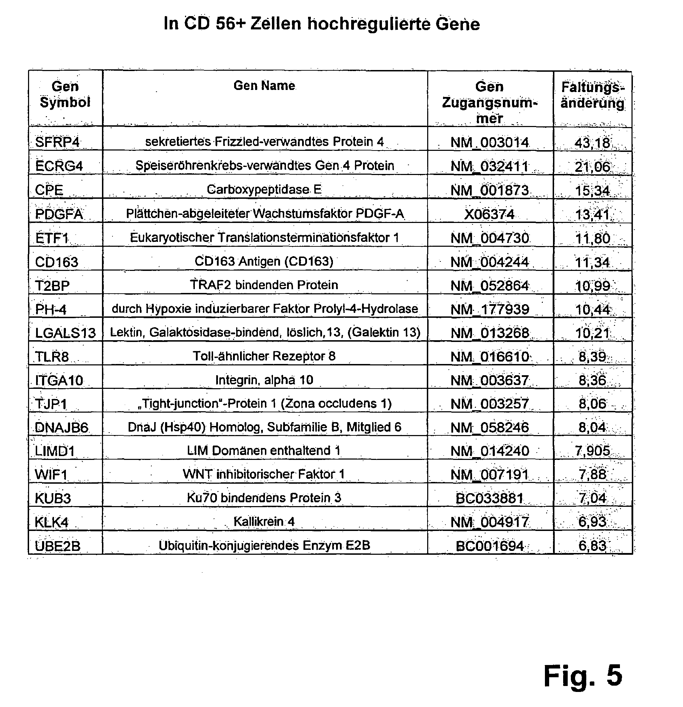

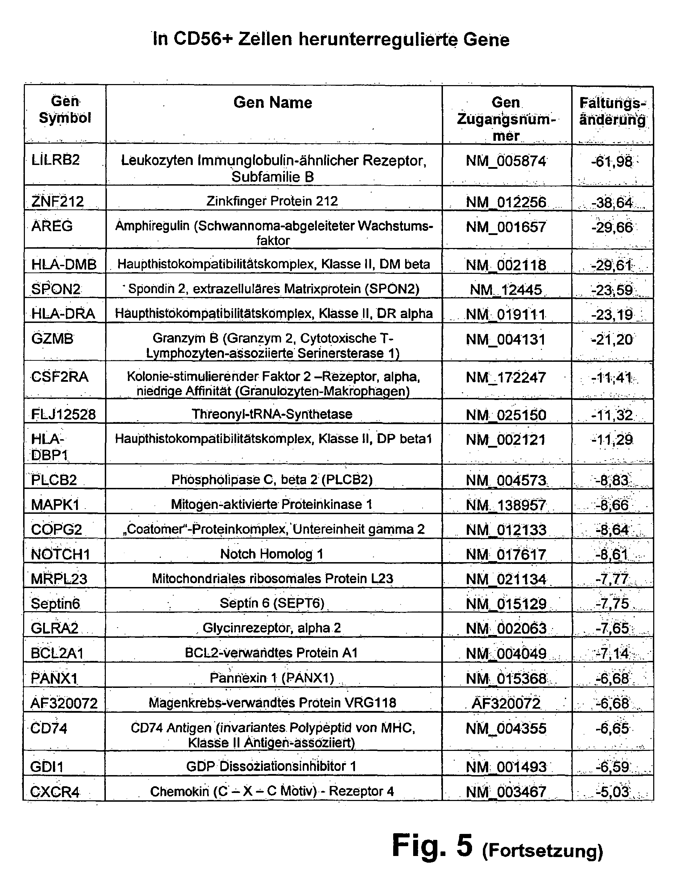

- Fig. 5: Tabelle 1: Microarray-Gen-Expressions-Analyse der CD271⁺CD56⁻ und CD271⁺CD56⁺ Knochenmarkszellen.
Die Mikroarray-Analyse wurde unter Verwendung von RNA aus 10.000 sortierten CD271⁺CD56⁻ und CD271⁺CD56⁺ Knochenmarkszellen durchgeführt. (A) Hochregulierte Gene in der CD271⁺CD56⁺ Population im Vergleich zu der CD271⁺CD56⁻ Fraktion. (B) Herunterregulierte Gene in der CD271⁺CD56⁺ Population im Vergleich zu der CD271⁺CD56⁻ Fraktion
- Fig. 6: Tabelle 2: Phänotyp und Proliferation der MSC-Klone (C1 bis C16) abgeleitet aus einzelnen TNAP⁺CD56⁻ und TNAP⁺CD56⁺ Knochenmarkszellen.
"+++" bedeutet >100 Durchschnittsfluoreszenzintensität (MFI); ""+" bedeutet 10 - 100 ΔMFI; "+" bedeutet 3 - 10 ΔMFI; "(+)" bedeutet 1 - 3 ΔMFI; "-" bedeutet eine negative Färbung.
- Fig. 7: Transfektion von HEK-293 Zellen mit TNAP:
Mit der kodierenden Sequenz von TNAP/ALPL (Homo sapiens alkaline phosphatase, liver/bone/ kidney (ALPL)) transfizierte HEK-293 Zellen, markiert mit W8B2-APC; die Zellen exprimieren neben TNAP (W8B2-Antigen) (A) auch das Reportergen GFP (B); und
- Fig. 8: Über den TNAP-spezifischen Antikörper W8B2 sortierte Knochenmarkzellen, gefärbt für den Nachweis der Alkalischen Phosphatase: W8B2-positive Zellen (A); W8B2-negative Zellen (B).

### Beispiele

### Material und Methoden

### Isolierung von Knochenmarkzellen und mononukleären peripheren Blutzellen

Knochenmark (bone marrow, "BM") wurde an der Berufsgenossenschaftlichen Klinik aus Oberschenkelschäften von Patienten gewonnen, die künstliche Hüftgelenke erhielten. Peripheres Blut (PB) von gesunden Spendern wurde vom Institut für Transfüsionsmedizin des Universitätsklinikums Tübingen erhalten. Mononukleare Zellen aus dem Knochenmark (BMMNC) und mononukleare Zellen aus dem Blut (PBMMC) wurden mittels Ficoll-Dichtegradienten-Fraktionierung isoliert,̅ und die verbleibenden Erythrocyten in einer Ammoniumchloridlösung lysiert.

### Kultur der Primärzellen

Die Ficoll-getrennten und FACS-angereicherten Knochenmarkzellen wurden folgendermaßen kultiviert: 2 x 10⁷ unfraktionierte oder 1 x 10⁴ sortierte TNAP⁺CD56⁺ und TNAP⁺CD56⁻ Knochenmarkzellen wurden in Gelatine-beschichteten T-75 oder T-25 Kulturflaschen in Anwesenheit von 20 ml oder 6 ml Knockout™ Ersatzmedium (Invitrogen, Karlsruhe, Deutschland) sowie 5 ng/ml rekombinantem humanem Fibroblasten-Wachstumsfaktor (rh-bFGF; CellSystems, Remagen, Deutschland) kultiviert. Nach einer dreitägigen Kultur wurden die nicht-adhärierenden Zellen entfernt und frisches Medium hinzugefügt. Die adhärenten Zellen wurden kultiviert, bis sie eine 90%-ige Konfluenz erreichten.

### Koloniebildender Fibroblast-Assay (CFU-F)

CFU-F-Assays wurden durch Ausplattieren von entweder 1 x 10⁵ unselektierten oder 500 - 5.000 FACS-selektierten BMMNC in Gelatine-beschichtete T-25 Flaschen durchgeführt, die Knockout™-Medium und 5 ng/ml rh-bFGF enthielten. Nach einer zwölftägigen Kultivierung wurden die adhärenten Zellen zweimal mit PBS gewaschen, fünf Minuten bei Raumtemperatur mit Methanol fixiert (Sigma-Aldrich), luftgetrocknet und mit Giemsa-Lösung gefärbt (Merck, Darmstadt, Deutschland). CFU-F-Kolonien wurden makroskopisch aufgezählt. Die Größe der Kolonien betrug zwischen 1 und 8 mm im Durchmesser.

### Differenzierung der MSC

*Osteoblasten- und Adipozytendifferenzierung:* MSC, die aus sortierten TNAP⁺CD56^{±} oder unfraktionierten BM-Zellen abgeleitet waren, wurden in NH OsteoDiff- oder NH AdipoDiff-Medium (Miltenyi Biotec, Bergisch Gladbach, Deutschland) kultiviert. Hierzu wurden 2 x 10⁴ (Osteogenese) oder 4 x 10⁴ (Adipogenese) MSC in 24-Well-platten kultiviert (Falcon, Heidelberg, Deutschland). Nach 12 Tagen in Kultur in NH OsteoDiff-Medium wurden die Zellen mit Methanol fixiert (-20 °C, 5 min). Die alkalische Phosphatase-Aktivität in Osteoblasten wurde unter Verwendung des FAST™ BCIP/NBT Substrats bestimmt (Sigma-Aldrich). Die Kalzium-Absetzung in fixierten Zellen (4 % PFA, 15 min) wurde nach Färben mit 2 % Alizarin Red (Merck) für 10 min bei Raumtemperatur analysiert. Die Bildung von Adipozyten wurde nach 25 Tagen in Kultur in NH AdipoDiff-Medium und nach einer 45-minütigen Färbung bei Raumtemperatur der Methanol-fixierten Zellen mit Oil Red O-Farbstoff (Sigma-Aldrich) untersucht. Bilder wurden unter Verwendung eines Axiovert 40C-Mikroskops (Carl Zeiss GmbH, Göttingen, Deutschland) gemacht.

*Chondrogene Differenzierung:* 4 x 10⁵ MSC wurden 4 Stunden lang bei 37 °C in 20 µl unvollständigem chondrogenem Induktionsmedium (PAA, Pasching, Österreich) kultiviert, das 1 % ITS-Supplemente (Sigma-Aldrich), 175 µM L-Ascorbinsäure (Sigma-Aldrich), 350 µM L-Prolin (Sigma-Aldrich), und 100 nM Dexamethason (Sigma-Aldrich) enthielt. Nach Inkubation wurden 400 µl des vollständigen chondrogenen Induktionsmediums, das mit 10 ng/ml an TGF-β₃ (Sigma-Aldrich) ergänzt war, hinzugefügt. Die resultierenden Zellpellets wurden 3 Wochen lang kultiviert, mit 4 % PFA fixiert, in Paraffin eingebettet, und in 5 µm dicke Schnitte geschnitten. Die getrockneten und deparaffinisierten Schnitte wurden mit Alcian Blau Lösung (Merck) für 45 min bei Raumtemperatur inkubiert, in 3 % Essigsäure gewaschen, eingebettet und mit einem Zeiss Axiovert 200 Mikroskop fotografiert.

*Myogene Differenzierung:* 5 x 10⁵ MSC wurden 7 Tage lang auf Gefäßen mit sehr niedriger Adhärenz in DMEM High Glucose (Invitrogen) kultiviert, das mit 100 µM β-Mercaptoethanol ergänzt war. Die resultierenden Cluster wurden in Gelatine-beschichtete 24-Well-Gefäße für 21 bis 28 Tage gegeben, die resultierenden Zellen mit 4 % PFA fixiert (45 min, bei Raumtemperatur), und mit 0,1 % Triton X-100/PBS über 20 min permeabilisiert. Die Zellen wurden über Nacht bei 4 °C mit Kaninchen-anti-humanen Antikörpern gegen Actin der glatten Muskulatur (SMA) (Spring Bioscience, Freemont, Kalifornien, USA) markiert, sowie mit einem Maus-anti-humanem Antikörper gegen Sarcomer-Actinin (anti-alpha-Actinin) (Sigma-Aldrich). Nach dem Waschen wurden die Zellen mit Cy3-konjugierten Ziege-anti-Kaninchen- IgG (Jackson Immuno Research) oder Alexa Fluor488-konjugierten Ziegen-anti-Maus- IgG (Invitrogen) sowie 0,4 µg/ml DAPI gefärbt.

*Neuronale Differenzierung:* 3,5 x 10⁴ MSC wurden 6 Tage lang in 800 µl NeüroCult^{®} NS-A-Proliferationsmedium (CellSystems) kultiviert, und anschließend 7 Tage lang in dem NeuroCult^{®} NS-A-Differenzierungsmedium (CellSystems). Die Zellen wurden mit 4 % PFA fixiert und mit 0,3 % Triton-X-100/PBS (Sigma-Aldrich) permeabilisiert, und zwar bevor sie über Nacht mit Kaninchen-anti-humanem Antikörper gegen das gliafibrilläre saure Protein (GFAP) oder dem Maus-anti-humanem Antikörper gegen das neuronale Klasse III-β-Tubulin (jeweils von CellSystems) inkubiert wurden. Nach Waschen mit 0,1 % BSA/TBS/Tween-20 (Sigma-Aldrich) wurden die Zellen mit dem Cy3-konjugierten sekundären Schwein-anti-Kaninchen-Antikörper gefärbt (30 min bei Raumtemperatur; Jackson Immuno Research, Cambridge, Großbritannien), oder mit dem Alexa Fluor^{®}488-konjugierten Ziegen-anti-Maus IgG sekundären Antikörper (Invitrogen), und 0,4 µg/ml DAPI.

*Pankreatische Differenzierung:* 5 x 10⁵ MSC wurden in 6-Napf-Gefäße mit sehr niedriger Adhärenz (Costar; CellSystems) ausplattiert, und 4 Tage lang in MEM kultiviert, das 1 mM Mono-Thioglycerol, 15 % ES-Cult FBS und 4,5 g/l DMEM High Glucose (Cell-Systems) enthielt. Die resultierenden Zellcluster wurden anschließend 6 Tage lang in 6-Napf adhärenten Platten (Falcon) in mit ITS-ergänztem, Serum-freiem Medium (CellSystems) kultiviert. Nach Überführen in Poly-L-Ornithin-beschichtete 24-Wellplatten wurden die Zellen 6 Tage lang in einem pankreatischen Proliferationsmedium (CellSystems) kultiviert, das N2-A und B27 Ergänzungsstoffe enthielt, sowie 25 ng/ml rh-bFGF, und dann anschließend für weitere 6 Tage in einem rh-bFGF-freien pankreatischen Differenzierungsmedium (CellSystems) mit 10 mM Nikotinamid. Nach dem Waschen wurden die Zellen mit 4 % PFA fixiert, mit 70 % Ethanol permeabilisiert und mit einem Blockierungspuffer inkubiert, der 0,25 % Triton X-100 und 2 % FBS enthielt. Anschließend wurden sie mit einem polyklonalen Kaninchen-anti-humanen Glucagon-Antikörper (1:75 Verdünnung; Dako Cytomations, Glostrup, Dänemark) oder mit einem polyklonalen Kaninchen-anti-human Insulinantikörper (1:200 Verdünnung; Anta Cruz Biotechnology) über Nacht markiert und mit einem sekundären Ziegen-anti-Kaninchen IgG-Cy³ (Millipore, Schwalbach, Deutschland) und 0,4 µg/ml DAPI gefärbt.

### Generierung der MSC-reaktiven monoklonalen Antikörper W8B2 und 39D5

Der monoklonale Antikörper W8B2, (IgG1; Spezifität für hTNAP) wurde durch Immunisierung von 6 bis 8 Wochen alten weiblichen Balb/c-Mäusen (Charles River WIGA, Sulzfeld, Deutschland) mit der Retinoblastom-Zelllinie WERI-RB-1 gewonnen. Der Antikörper 39D5 (IgG1, CD56) wurde durch Immunisierung mit der hämatopoetischen Zelllinie KG-1a gewonnen.

### Immunfluoreszenzanalyse und Zellsortierung

*Antikörper:* Die folgenden Antikörper wurden eingesetzt: 97C5 (CD10), 46A11 (CD13), 39D5 (CD56), 1G2C2 (CD105), 104D2 (CD117), W6B3C1 (CD133), 28D4 (CD140b), 67D2 (CD164), CUB1 (CD318; CDCP1), 24D2 (CD340; HER-2), W3C4E11 (CD349; frizzled-9), HEK-3D6 (unbekannt), W1C3 (unbekannt), W5C4 (unbekannt), W5C5 (unbekannt), W3D5 (unbekannt), und W8B2B10 (TNAP). CD34-PE (Klon 8G12), CD45-PE (Klon HI30), CD56-FITC (Klon NCAM16.2), CD56-PE (Klon NCAM16.2), CD90-APC (5E10), CD63-PE (Klon H5C6), CD73-PE (Klon AD2), und HLA-DR-PE (Klon TÜ36) wurden von Becton Dickinson (Heidelberg, Deutschland) gekauft. Der SSEA-4-reaktive Antikörper MC-813-70 wurde von Chemicon (Hampshire, Großbritannien) erworben. CD271-APC (Klon ME20.4-1.H4) wurde von Miltenyi Biotec erworben. CD105-PE (Klon SN6) wurde von eBioscience Inc. (San Diego, Kalifornien, USA) erworben. CD166-PE wurde von Dr. Gene Lay (BioLegend, San Diego, Kalifornien, USA) erhalten.

*Immunofluoreszenzfärbung:* Nach Blockierung und spezifischen Bindungen mit 10 mg/ml Polyglobin (10 min, 4 °C) wurden die Zellen 15 min lang mit entweder 20 µl Antikörpern oder 10 µl Fluorochrom-konjugierten Antikörpern inkubiert. Die mit den Konjugaten gefärbten Zellen wurden zweimal gewaschen, in 200 µl FACS Puffer suspendiert und für die Durchflusszytometrie eingesetzt. Die Zellen, die mit den Antikörpern markiert waren, wurden mit 20 µl eines F(ab)₂-Fragment des R-Phycoerythrin (PE)-konjugierten Ziegen-anti-Maus-Antikörpers (Dako Cytomations, Glostrup, Dänemark) 15 min lang gefärbt, zweimal gewaschen und mittels Durchfusszytometrie analysiert. Für die Vielfarbenfärbung wurden die Zellen 15 min lang mit 10 µl eines Anti-CD56-FITC und Anti-CD271-APC und/oder dem erwähnten PE-Konjugat inkubiert. Nach dem Waschen wurden die Zellen für die Durchfusszytometrie eingesetzt. Für eine kombinierte indirekte und direkte Färbung wurden die Zellen zunächst mit dem nicht-konjugierten Antikörper markiert, und anschließend mit 20 µl an 1:25 verdünntem Ziegen-anti-Maus sekundärem Antikörper 15 min lang gefärbt. Die freien Bindestellen des sekundären Antikörpers wurden durch Inkubation der Zellen für 25 min mit 20 µl an einem Maus-IgG-polyklonalen Antikörper (0,05 µg/ml; Southern Biotech, Birmingham, AL) blockiert, bevor sie mit CD271-APC und/oder CD56-FITC gegengefärbt wurden. Nach einem Waschschritt wurden die Zellen mittels Durchfusszytometrie analysiert.

### Duchflusszytometrische Analysen und Zellsortierung

Die Zellen wurden auf einem FACSAria Zellsortierer (Becton Dickinson) sortiert, oder mit einemFACSCantoII-Flusszytometer (Becton Dickinson) analysiert. Die Daten wurden unter Verwendung der FCS-Express-Software (De Novo Software, Ontario, Kanada) analysiert. Die Einzelzellsortierung in 96-Napfplatten wurde unter Verwendung der ACDU-Vorrichtung durchgeführt.

### MACS-Trennung

In ausgewählten Versuchen wurden die Knochenmarkszellen mittels MACS (Miltenyi Biotec) unter Verwendung von CD271-APC und Anti-APC Beads vorsortiert. Die Trennungen wurden gemäß den Empfehlungen des Herstellers durchgeführt.

### Gen-Chip-Analyse der sortierten Zellen

Zehntausend TNAP⁺CD56⁻ und TNAP⁺CD56⁺ Zellen wurden für eine kommerzielle Gen-Chip-Analyse (Miltenyi Biotec) eingesetzt, um ein Oligo-Microarray des humanen Gesamtgenoms (Agilent Technologies, Böblingen, Deutschland) durchzuführen. Die amplifizierten cDNAs wurden unter Verwendung eines ND-1000 Spektrophotometers (NanoDrop Technologies Inc., Wilmington, DE) quantifiziert. 250 ng der Bibliothek-PCRs wurden als Template für Cy3- und Cy5-Markierung eingesetzt. Die Proben wurden 17 Stunden lang bei 65 °C auf dem Microarray von Agilent gemäß den Anleitungen des Herstellers hybridisiert. Das Scannen des Gen-Chips und die Datenanalyse wurden unter Verwendung der Luminator-Software (Miltenyi Biotec) durchgeführt.

### Ergebnisse

Der monoklonale Antikörper 39D5 erkennt ein Epitop von CD56, das nicht auf der Oberfläche von peripherem Blut (PB)-abgeleiteten NK-Zellen exprimiert wird. Eine vergleichende Durchflusszytometrie-Analyse zeigte, dass nur der kommerziell erhältliche CD56-spezifische Antikörper NCAM16.2, jedoch nicht der monoklonale Antikörper 39D5, mit 20 ± % der PB-Zellen reagierte (Fig. 1A). Jedoch reagierten beide Antikörper mit einer kleinen Subpopulation von BM CD271^{bright}-Zellen (Fig. 1B). Eine simultane Färbung von BM-Zellen mit 39D5 und NCAM16.2 ergab, dass beide Antikörper die gleiche CD271^{bright}-Population detektierten.

### Die CD271^{bright} CD56⁺-Population ist für CFU-F angereichert

Um das klonogene Potential sortierter CD56⁺ und CD56⁻ Subsets zu bestimmen, wurden CFU-F-Assays durchgeführt. Die Fig. 1C zeigt eine dreifach (± 0,8) angereicherte Effizienz von CD271^{bright}CD56⁺ Zellen im Vergleich zu CD271^{brightt}CD56⁻ Zellen und eine 180-fache (± 52) Anreicherung an CFU-F im Vergleich zu den unfraktionierten BM-Zellen. Die Anreicherung war unabhängig von dem analysierten CD56 Epitop. Interessanterweise erzielten die CD271^{bright}CDS6⁺ Zellen nicht nur höhere Koloniezahlen (38/500 gegenüber 12/500 plattierten Zellen), sondern waren auch 2-bis 4-fach angereichert in den sehr großen Kolonien (>100 Zellen/Kolonie).

### Phänotyp der MSC, die von den sortierten CD271^{bright}CD561^{±}-BM-Zellen abgeleitet waren

CD271^{bright}CD56⁺ und CD271^{bright}CD56⁻ Zellen wurden mittels FACS getrennt, in Gelatine-beschichtete Flaschen in Gegenwart eines Serum-Ersatzmediums (n = 3) kultiviert, mit den angegebenen Antikörpern gefärbt und durchflusszytometrisch analysiert. Fig. 1D zeigt, dass CD10, CD140b, CD318, HER2 (CD340), frizzled-9 (CD349), ebenso wie die Antikörper-definierten Antigene W1C3, W5C4, W5C5 und W3D5 in ähnlicher Weise auf MSC exprimiert waren, die von beiden Fraktionen abgeleitet waren. CD271, SSEA-4 und CD56 waren am dichtesten exprimiert auf CD271^{bright}CD56⁺-abgeleiteten MSC, wohingegen die TNAP (W8B2-Antigen)-Expression auf CD271^{bright}CD56⁻ abgeleiteten MSC ausgeprägter war. Im Gegensatz zu primären MSC (Fig. 2B) exprimierten kultivierte MSC *de novo* CD 166 und CD318, und regulierten die CD271-Expression herunter.

### Genexpressions-Analyse primärer CD271^{bright}CD56⁻ und CD271^{bright}CD56⁺ Knochenmarkszellen

Eine Microarray-Analyse des Gesamtgenoms von 10.000 sortierten Knochenmarkszellen wurde durchgeführt, um das Genexpressions-Profil von CD271^{bright}CD56⁻ und CD271^{bright}CD56⁺ Knochenmarkszellen zu vergleichen. CD271^{bright}CD56⁺-Zellen zeigten eine 11- bis 43-fach erhöhte Expression des sekretierten frizzled-verwandten Protein 4, des Speiseröhrenkrebs-bezogenen Gen-4-Protein, der Carboxypeptidase E, des Plättchen-abgeleiteten Wachstumsfaktors A, des eukaryotischen Translations-Terminationsfaktors 1 und von CD163 (Fig. 5: Tabelle 1A). Im Gegensatz dazu waren die Gene, die für die Leukozytenimmunglobulin-ähnliche Rezeptorsubfamilie B kodierten, für das Zinkfingerprotein 212, Amphiregulin, HLA-Klasse II DM beta, spondin 2 und HLA-Klasse II DR alpha mit 62- bis 23-fach erniedrigten Leveln in diesem Subset exprimiert (Tabelle 1B), was auf eine hohe Diversität der Genexpressionsprofile in diesen Subsets hindeutet.

### Phänotyp der CD271^{bright}CD56^{±} Knochenmarkszellen

Um die Expressionsprofile der Oberflächenmarker auf CD271^{bright}CD56⁺ und CD271^{bright}CD56⁻ Zellen zu vergleichen, wurden die Knochenmarkszellen dreifach mit Anti-CD271, Anti-CD56 und einer Reihe von Testantikörpern gefärbt und auf die CD271^{bright}-Population eingegrenzt (Fig. 2A; Fenster R1). Die Fig. 2B zeigt, dass CD63, CD73, CD140b, CD164 und das W3D5-Antigen mit einem ähnlichen Level auf beiden Zellsubsets exprimiert waren, wohingegen CD45, CD117, CD133, CD318 negativ waren. Im Gegensatz dazu exprimierten CD271^{bright}CD56⁺ Zellen CD13, CD105, frizzled-9 (CD349), HLA-DR und TNAP (W8B2 Antigen) mit einem reduzierten Level, wohingegen CD166 exklusiv auf diesen Zellen gefunden wurde. Die Tatsache, dass die CD166-Expression auf den Großteil der primären MSC fehlt, überraschte, da kultivierte MSC bekanntermaßen hohe Level an CD166 exprimieren. Auch das Tumorantigen CDCP1 (CD318) war auf primärem CD271^{bright}CD56⁻ und CD271^{bright}CD56⁺ Zellen negativ (Fig. 2B), jedoch in kultivierten MSC stark exprimiert (Fig. 1B).

### Klonogene Fähigkeit von TNAP⁺CD56^{±} Knochenmarkszellen

Mit den vorliegenden Ergebnissen konnte gezeigt werden, dass TNAP mit einem hohen Level auf CD271^{bright}CD56⁻ Zellen exprimiert ist und mit einem niedrigeren Level auf CD271^{bright}CD56⁺ Zell-Subsets (Fig. 2A). Um die klonogene Fähigkeit dieser Subsets zu untersuchen, wurden die Zellen mit den Fenstern R2 und R3 fraktioniert (Fig. 2C). Definierte Zellzahlen wurden in Kulturflaschen gegeben, und die resultierenden CFU-F nach einer 12-tägigen Kultivierung ausgezählt. Die Fig. 2D zeigt, dass TNAP⁺CD56⁺-Zellen zu einer 2- (± 0,4) -fach höheren CFU-F-Anzahl führten als die TNAP⁺CD56⁻ Zellen. Eine Giemsa-Färbung zeigte, dass die TNAP⁺CD56⁻ Zellen ein großes und helles Zytoplasma mit Vacuolen enthielten, wohingegen die TNAP⁺CD56⁺ Zellen ein kleineres Zytoplasma mit basophilen Körperchen enthielten (Fig. 2E).

### Differenzierungsfähigkeit von MSC, abgeleitet aus TNAP⁺CD56^{±}-Zellen

Für die Differenzierungsassays wurden unfraktionierte oder sortierte TNAP⁺CD56⁻, TNAP⁺CD56⁺ Zellen expandiert, bis sie ungefähr 9 bis 10 Zellteilungen unterzogen waren. Eine definierte Anzahl der resultierenden MSC wurde anschließend induziert, um in Zellen der osteogenen, adipogenen, chondrogenen, myogenen, neuronalen und pankreatischen Linien zu differenzieren.

*Osteoblästische Differenzierung:* Die Kultur von MSC, die aus sortierten Zell-Subsets abgeleitet waren, in einem geeigneten Medium führte zu einem Auftreten von 95 ± 5 % (CD56⁺) und 70 ± 5 % (CD56⁻) an alkalische-Phosphatase-positiven Zellen (Fig. 3). Im Gegensatz dazu führten MSC, die von den unfraktionierten Knochenmarkszellen abgeleitet waren, zu lediglich 35 % ± 5 % an alkalische-Phosphatase-positiven Zellen. Eine Alizarin Rot S-Färbung war in sämtlichen Osteoblastenfraktionen zu beobachten. Jedoch war die Anzahl der Kalziumablagerung in den Osteoblasten, die aus den unfraktionierten Zellen abgeleitet waren, um das Zweifache höher.

*Adipozyten-Differenzierung:* Die Kultivierung der unfraktionierten und TNAP⁺CD56⁻-abgeleiteten MSC in Adipozyten-Differenzierungsmedium führte zu einem Auftreten von Oil Red O-einbauenden Adipozyten. Im Gegensatz dazu waren TNAP⁺CD56⁺-abgeleitete MSC nicht in der Lage, Adipozyten zu bilden (Fig. 3). TNAP⁺CD56⁻ MSC zeigten einen 5 ± 0,5-fachen Anstieg in Oil Red O-positiven Adipozyten, im Vergleich zu unfraktionierten Zellen. Daher ist die Fähigkeit zur Adipozyten-Differenzierung auf das TNAP⁺CD56⁻ Subset beschränkt.

*Chondrogene Differenzierung:* Um das Potential zur Chondrozyten-Differenzierung zu analysieren, wurden MSC, die aus den fraktionierten und unfraktionierten Zellen abgeleitet waren, in einem geeigneten Medium kultiviert und die resultierenden Zellpellets mit Alcian Blau gefärbt. Obgleich eine chondrogene Differenzierung in beiden Fraktionen detektiert wurde, waren Pelletschnitte aus TNAP⁺CD56⁺ Zellen 5 (± 1,6) mal größer als die aus TNAP⁺CD56⁻ Zellen (Fig. 3). Darüber hinaus wurden lebensfähige Chondrozyten beinahe ausschließlich im TNAP⁺CD56⁺ Subset detektiert, wohingegen TNAP⁺CD56⁻ Pellets hauptsächlich apoptotische Zellen enthielten. MSC aus unfraktionierten Zellen führten zu heterogenen Pelletgrößen, jedoch durchgehend mit weniger lebensfähigen Zellen. Diese Daten legen nahe, dass eine effektive Chondrogenese auf das TNAP⁺CD56⁺ MSC Subset beschränkt ist.

*Myogene Differenzierung:* Die Kultur der TNAP⁺CD56^{±}-abgeleiteten MSC in einem Medium, das für die Differenzierung in Zellen der gestreiften Muskulatur bestimmt war, führte zu einem Auftreten einer für die gestreifte Muskulatur spezifische α-Actininfärbung in Zellen aller Fraktionen (Fig. 3). Im Gegensatz dazu war der für die glatte Muskulatur spezifische Marker SMA in allen Fraktionen negativ. Undifferenzierte MSC zeigten eine sehr schwache α-Actininfärbung.

*Neuronale Differenzierung:* MSC, die in einem neuronalen Differenzierungsmedium kultiviert wurden, wurden hinsichtlich GFAP und neuronaler β-Tubulin-III gefärbt. Fig. 3 zeigt eine deutliche Färbung von Zellen, die aus unfraktionierten und aus TNAP⁺CD56⁺ und TNAP⁺CD56⁻-abgeleiteten MSC abgeleitet waren. In undifferenzierten MSC oder in differenzierten Zellen, die mit Isotyp-spezifischen Kontrollantikörpern markiert waren, wurde keine Färbung beobachtet.

*Pankreatische Differenzierung:* Die Kultur von MSC in einem pankreatischen Differenzierungsmedium führte zu einer Glucagon- und Insulin-Färbung Pankreas-ähnlicher Inseln in Zellen sämtlicher Fraktionen (Fig. 3). Jedoch waren die Inseln, die aus TNAP⁺CD56⁺ MSC abgeleitet waren, größer, und die Färbungsintensität dieser Marker war deutlich ausgeprägter im Vergleich zu TNAP⁺CD56⁻-abgeleiteten oder unfraktionierten MSC. In den undifferenzierten MSC oder in den differenzierten Zellen, die mit einem Isotyp-passenden Kontroll-Antikörper markiert waren, wurde keine Färbung beobachtet.

### Einzelzellanalyse der TNAP⁺CD56⁻ Klone

Die Wachstumscharakteristika, der Phänotyp und die Differenzierungsfähigkeit einzelner TNAP⁺CD56⁺ und TNAP⁺CD56⁻ Zellen wurde bestimmt, indem einzelne Zellen in Gelatine-beschichtete 96-Wellkulturplatten sortiert,̅ und in einem Serumfreien Medium kultiviert wurden, bis makroskopisch sichtbare Kolonien (>20 Zellen) auftraten. Die Klonierungseffizienz der sortierten TNAP⁺CD56⁺ und TNAP⁺CD56⁺ Zellen betrug, 11/96 respektive 5/96. Diese ca. zweifach erhöhte Frequenz der CD56⁺ Zellen steht in Übereinstimmung mit den zweifach höheren Koloniewerten der in Fig. 2D beschriebenen sortierten Zellen.

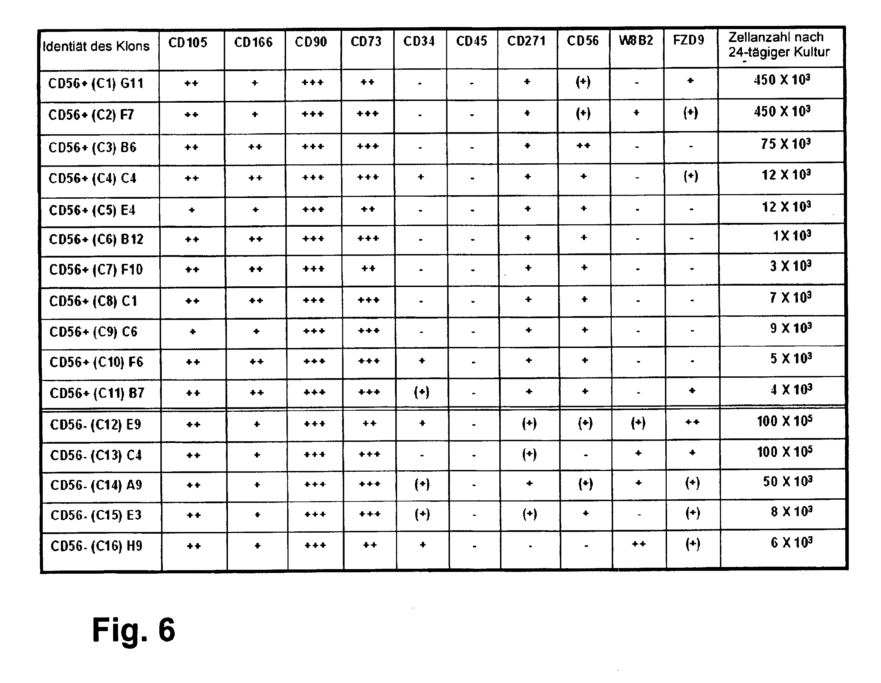

Die resultierenden Kolonien wurden in T-25 Flaschen überführt und expandiert, bis sie 60 bis 70 % Konfluenz erreichten. Eine Phänotyp-Analyse zeigte, dass sämtliche 16 Klone negativ für CD45 waren, jedoch CD73, CD90, CD105 und CD166 exprimierten, und sie zeigten ferner eine reduzierte CD271 Expression (Fig. 4A, Fig. 6: Tabelle 2). Sämtliche CD56⁺ Klone - mit Ausnahme des Klons C3 - regulierten die CD56-Expression herunter. Im Gegensatz dazu war die CD56-Expression in zwei von vier MSC-Klonen, die aus CD56⁻ Zellen abgeleitet waren, induziert (C14, C15). Interessanterweise konnte eine signifikante CD34-Expression in zwei der CD56⁺- und CD56⁻ Klone beobachtet werden, wohingegen TNAP nur in einem CD56⁺ Klon und in vier von fünf CD56⁻ Klonen detektiert wurde. Auch die Frizzled-9-Expression wurde lediglich in vier von elf CD56⁺ Klonen beobachtet, sowie in sämtlichen CD56⁻ Klonen. Diese Daten zeigen, dass jeder einzelne Klon ein individuelles Expressionsprofil besitzt, mit einer bevorzugten Expression von TNAP und Frizzled-9 in den CD56⁻ Klonen.

Die Expansion aller 16 einzelnen Zellen über 24 Tage führte zu einem Auftreten spindelförmiger Zellen mit einer Fibroblasten-ähnlichen Morphologie (Fig. 4B). Unter den einzelnen Klonen wurde hinsichtlich des Proliferationspotentials eine starke Heterogenität beobachtet (Fig. 6: Tabelle 2). Obgleich die durchschnittliche Anzahl der Zellen, die von den CD56⁺ Klonen abgeleitet waren, ungefähr zweimal so hoch war wie die von den CD56⁻ Klonen abgeleiteten Zellen (93,5 x 10³ gegenüber 52,8 x 10³ nach einer 24-tägigen Kultur) konnte kein Zusammenhang zwischen den einzelnen Klonen oder den Phänotyp-Profilen detektiert werden (Fig. 6: Tabelle 2).

Die stark proliferierenden Klone C1 und C2 (TNAP⁺CD56⁺) und C12 und C13 (TNAP⁺CD56⁻) wurden auch hinsichtlich ihres osteoblastären, adipozytären und neuronalen Differenzierungspotential analysiert. Aus Fig. 5C ist zu entnehmen, dass lediglich ein CD56⁻ Klon, jedoch keiner der CD56⁺ Klone zur Entstehung von Oil Red O-Färbstoff-einbauenden Adipozyten führte. Alkalische Phosphatase-positive Osteoblasten und neuronales β-Tubulin-III exprimierende Neuronen-ähnliche Zellen wurden von drei von vier Klonen generiert, jedoch nicht durch den CD56⁻ Klon C13 (Fig. 4C). Bemerkenswert war, dass β-Tubulin-III-positive Zellen in den CD56⁺ Klonen 5-10-mal häufiger waren als in den CD56⁻ Klonen.

### Transfektion von HEK-293 Zellen mit TNAP

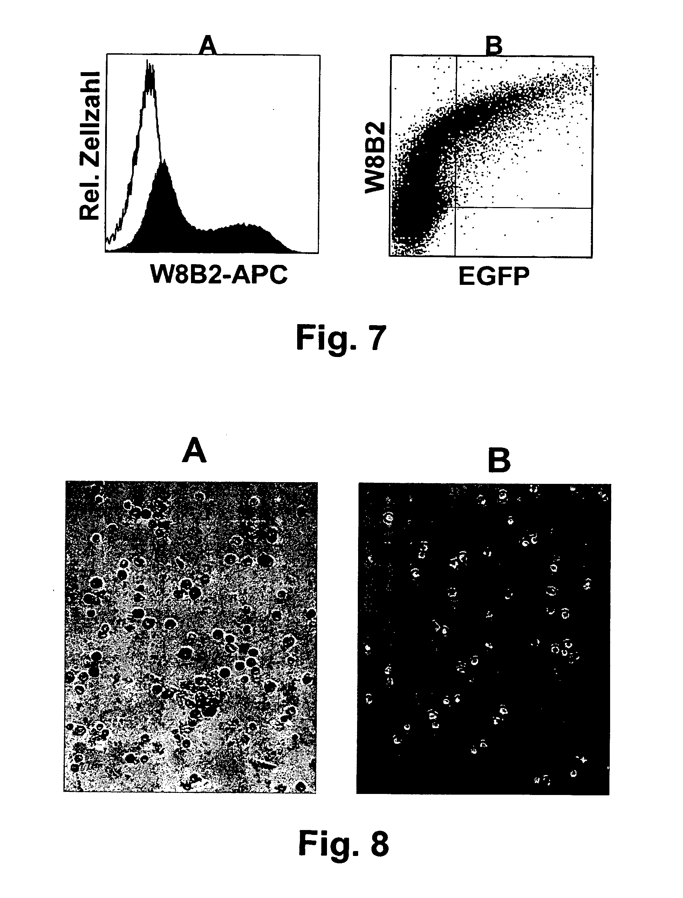

HEK-293 Zellen (erhältlich bei der Deutschen Sammlung für Mikroorganismen und Zellkulturen, DSMZ unter der Nummer DSMZ Nr. ACC 305) wurden mit der kodierenden Sequenz von TNAP/ALPL (Homo sapiens alkaline phosphatase, liver/bone/ kidney (ALPL)) transfiziert, die in den Vektor pCMV6-AC-GFP (erhältlich bei der Firma Origene Technologies, Rockville, MD, USA) integriert wurde. Zur Transfektion wurde das Reagenz MegaTran1.0 transcript 1 (ebenfalls OriGene Technologies, Rockville, MD, USA) verwendet. Nach der Inkubation wurden die Zellen gewaschen und mit W8B2-APC markiert. Die durchfluss-zytometrische Analyse (siehe Fig. 7) zeigt, dass ca. 40% der Zellen stark positiv für W8B2 sind: Die Zellen exprimieren neben TNAP (W8B2-Antigen) (Fig. 7A) auch das Reportergen Green-Fluorescent Protein (Grün fluoreszierendes Protein (GFP)) (Fig. 7B).

### Nachweis der alkalischen Phosphatase-Aktivität

Knochenmarkzellen wurden mit dem TNAP-spezifischen Antikörper W8B2 markiert (W8B2-APC) und im FACSAria (BD Biosciences, Franklin Lakes, NJ USA) Zellsortiergerät sortiert. Die sortierten Zellen wurden anschließend mit einem kommerziell erhältlichen Kit für den Nachweis der Alkalischen Phosphatase gefärbt (StemTAG AP staining kit; Stem Cell Technologies, Vancouver, CA). Nur W8B2-positive Zellen zeigten eine Reaktion für dieses Enzym (Fig. 8A). W8B2-negative Zellen zeigten keine Reaktion (Fig. 8B).

Mit den vorliegenden Ergebnissen wurden MSC-Populationen charakterisiert, die von CD56-spezifischen und TNAP-spezifischen Antikörpern erkannt werden. Bemerkenswert war, dass eine effektive Chondrozyten- und Pankreas-ähnliche Insel-Differenzierung nur in Fällen der TNAP⁺CD56⁺ Fraktion induziert werden konnte. Im Gegensatz dazu konnten Adipozyten lediglich aus TNAP⁺CD56⁻ Zellen generiert werden. Das Aussortieren einzelner Zellen aus beiden Subsets bestätigte die unterschiedliche Proliferations- und Differenzierungsfähigkeit der TNAP⁺CD56⁺ und TNAP⁺CD56⁻ Zellen.

Mit den vorliegenden Ergebnissen konnte auch gezeigt werden, dass nur die TNAP⁺CD56⁺ Zellen dazu in der Lage waren, effektiv in Chondrozyten zu differenzieren, wie durch die erhöhte Knorpelpelletgröße und die extensive Proteoglykanfärbung gezeigt werden konnte.

Mit der vorliegenden Studie wurden daher Antigene identifiziert, nämlich TNAP und CD56, über welche effektiv MSC mit chondrogenem, adipozytärem oder pankreatischem Differenzierungspotential identifiziert und/oder isoliert werden können.

Diese Ergebnisse sind insbesondere im Hinblick auf den klinischen Einsatz der derart isolierten Stammzellen bzw. der über diese Stammzellen gewonnenen Chondrozyten/Adipozyten/Pankreas-ähnlichen Zellen relevant. So sind bspw. Verletzungen des Gelenkknorpels und von Bandscheiben regelmäßig schwierig zu behandeln, gerade aufgrund der begrenzten Regenerationsfähigkeit dieser Gewebe. Krankheiten wie rheumatoide Arthritis, Traumata, Knochenbrüche und Bandscheibenverletzungen sind direkt mit dem Mangel einer effektiven Chondrogenese verbunden. Trotz des Fortschritts in der Orthopädie und des steigenden Erfolgs bei der autologen Chondrozytentransplantation bleiben Zellbiologie-bezogene Ansätze für die Knorpelregeneration eine Herausforderung. Das Hauptproblem ist die Verwendung kultivierter Zellen für klinische Zwecke, bei welchen die Ausgangszellen lediglich schlecht charakterisiert sind.

Daher bietet die vorliegende Erfindung die Möglichkeit, hoch angereicherte und gut definierte TNAP⁺CD56⁺ Knochenmarkszellen bereitzustellen, mit einer herausragenden chondrogenen Differenzierungsfähigkeit, die für den klinischen Einsatz als Ausgangskultur bzw. Population eingesetzt werden können. Diese Zellen können entweder direkt zur Injektion eingesetzt werden, bspw. in die Bandscheibenzwischenräume/Bandscheiben, oder *in vitro* expandiert und in Chondrozyten differenziert werden, bevor sie für klinische Einsätze verwendet werden.

## Patentansprüche

1. In vitro Verwendung eines Antikörpers oder funktionellen Fragmenten des Antikörpers, der an das das Antigen TNAP bindet, in Kombination mit einem Antikörper oder funktionellen Fragmenten des Antikörpers, der an CD56 bindet, zur Isolierung von Stammzellen mit adipozytärem, chondrozytärem und pankreatischem Differenzierungspotential.

2. Verwendung nach Anspruch 1, **dadurch gekennzeichnet, dass** der anti-TNAP-Antikörper ausgewählt ist aus der Gruppe:
- der Antikörper W8B2, der von der bei der Deutschen Sammlung von Mikroorganismen und Zellkulturen hinterlegten Zelllinie mit der Nr. ACC 2567 produziert wird,
- funktionelle Fragmente des Antikörpers W8B2, die an das Antigen TNAP binden, der von der bei der Deutschen Sammlung von Mikroorganismen und Zellkulturen hinterlegten Zelllinie mit der Nr. ACC 2567 produziert wird, und
- ein Antikörper, der an das gleiche Epitop bindet wie der Antikörper W8B2, der von der bei der Deutschen Sammlung von Mikroorganismen und Zellkulturen hinterlegten Zelllinie mit der Nr. ACC 2567 produziert wird.

3. Verwendung nach Anspruch 2, **dadurch gekennzeichnet, dass** der an CD56 bindende Antikörper ausgewählt ist aus der Gruppe:
- der Antikörper 39D5, der von der bei der Deutschen Sammlung von Mikroorganismen und Zellkulturen hinterlegten Zelllinie mit der Nr. ACC 2930 produziert wird,
- funktionelle Fragmente des Antikörpers 39D5, die an CD56 binden, der von der bei der Deutschen Sammlung von Mikroorganismen und Zellkulturen hinterlegten Zelllinie Nr. ACC 2930 produziert wird,
- ein Antikörper, der an das gleiche Antigen bindet wie der Antikörper 39D5, der von der bei der Deutschen Sammlung von Mikroorganismen und Zellkulturen hinterlegten Zelllinie Nr. ACC 2930 produziert wird, und
- ein Antikörper, der an das gleiche Epitop bindet wie der Antikörper 39D5, der von der bei der Deutschen Sammlung von Mikroorganismen und Zellkulturen hinterlegten Zelllinie Nr. ACC 2930 produziert wird.

4. Verwendung nach einem der Ansprüche 1 bis 3, **dadurch gekennzeichnet, dass** zu der Kombination aus einem anti-TNAP und einem gegen CD56 gerichteten Antikörper zusätzlich noch ein gegen CD271 gerichteter Antikörper, oder funktionelle Fragmente des Antikörpers, die an CD271 binden, eingesetzt wird.

5. Verfahren zur Isolierung und/oder Identifizierung von mesenchymalen Stammzellen mit adipozytärem Differenzierungspotential, **dadurch gekennzeichnet, dass** es die folgenden Schritte aufweist:
a) in-Kontakt-Bringen einer Probe, die mesenchymale Stammzellen enthält, mit einem Antikörper oder mit funktionellen Fragmenten des Antikörpers, der an das Antigen TNAP bindet, ,
b) in-Kontakt-Bringen der Probe aus Schritt a) mit einem Antikörper, der an CD56 bindet, oder mit funktionellen Fragmenten des Antikörpers, die an CD56 binden, und
c) Isolieren und/oder Identifizieren von Zellen, an welche der Antikörper oder funktionelle Fragmente des Antikörpers, der an das Antigen TNAP bindet, nicht aber der Antikörper oder funktionelle Fragmente des Antikörpers, der an CD56 bindet, gebunden hat.

6. Verfahren zur Isolierung und/oder Identifizierung von mesenchymalen Stammzellen mit chondrozytärem Differenzierungspotential, **dadurch gekennzeichnet, dass** es die folgenden Schritte aufweist:
a) in-Kontakt-Bringen einer Probe, die mesenchymale Stammzellen enthält, mit einem Antikörper oder mit funktionellen Fragmenten des Antikörpers, der an das Antigen TNAP bindet,
b) in-Kontakt-Bringen der Probe aus Schritt a) mit einem Antikörper oder mit funktionellen Fragmenten des Antikörpers, der an CD56 bindet, und
c) Isolieren und/oder Identifizieren von Zellen, an welche sowohl der Antikörper oder funktionelle Fragmente des Antikörpers, der an das Antigen TNAP bindet, als auch der Antikörper oder funktionelle Fragmente des Antikörpers, der an CD56 bindet, gebunden haben.

7. Verfahren nach Anspruch 6, **dadurch gekennzeichnet, dass** es den weiteren Schritt c') aufweist:
c') in-Kontakt-Bringen der Probe aus Schritt b) mit einem Antikörper oder mit funktionellen Fragmenten des Antikörpers, der an CD271 bindet.

8. Verfahren nach einem der Ansprüche 5 bis 7, **dadurch gekennzeichnet, dass** der in Schritt a) eingesetzte anti-TNAP-Antikörper ausgewählt ist aus der Gruppe:
- der Antikörper W8B2, der von der bei der Deutschen Sammlung von Mikroorganismen und Zellkulturen hinterlegten Hybridomzelllinie mit der Nr. ACC 2567 produziert wird,
- funktionelle Fragmente des Antikörpers W8B2, die an das Antigen TNAP binden, und
- Antikörper, die an das gleiche Epitop binden wie der Antikörper W8B2, der von der bei der Deutschen Sammlung von Mikroorganismen und Zellkulturen hinterlegten Hybridomzelllinie mit der Nr. ACC 2567 produziert wird.

9. Verfahren nach einem der Ansprüche 5 bis 8, **dadurch gekennzeichnet, dass** der in Schritt b) eingesetzte an CD56 bindende Antikörper ausgewählt ist aus der Gruppe:
- der Antikörper 39D5, der von der bei der Deutschen Sammlung von Mikroorganismen und Zellkulturen hinterlegten Hybridomzelllinie Nr. ACC 2930 produziert wird,
- funktionelle Fragmente des Antikörpers 39D5, die an CD56 binden, und
- Antikörper, die an das gleiche Epitop binden wie der Antikörper 39D5, der von der bei der Deutschen Sammlung von Mikroorganismen und Zellkulturen hinterlegten Hybridomzelllinie Dr. ACC 2930 produziert wird.

10. Verfahren nach einem der Ansprüche 5 bis 9, **dadurch gekennzeichnet, dass** die Schritte a), b) und c') gleichzeitig, aufeinanderfolgend oder in umgekehrter Reihenfolge durchgeführt werden.

11. TNAP⁺ CD56⁺ -Stammzellen, die mit einem Verfahren nach einem der Ansprüche 6 und 7 oder 8 oder 9 oder 10 isoliert und/oder identifiziert wurden zur Anwendung für die Therapie oder Diagnostik.

12. Verwendung von TNAP⁺ CD56⁺ -Stammzellen, die mit einem Verfahren nach einem der Ansprüche 6 und 7 oder 8 oder 9 oder 1.0 isoliert und/oder identifiziert wurden, zur definierten Generierung von Chondrozyten und Pankreasähnlichen Zellen.

13. Verwendung nach Anspruch 12, **dadurch gekennzeichnet, dass** die TNAP⁺ CD56⁺ -Stammzellen, die mit einem Verfahren nach einem der Ansprüche 6 bis 10 isoliert und/oder identifiziert und zu Chondrozyten und Pankreasähnlichen Zellen differenziert wurden, zur Therapie und/oder Prophylaxe eingesetzt werden können.

14. TNAP⁺ CD56⁺ -Stammzellen, die mit einem Verfahren nach einem der Ansprüche 6 und 7 oder 8 oder 9 oder 10 isoliert und/oder identifi-ziert wurden zur Anwendung für die Therapie und/oder Prophylaxe von Knorpel-, Knochen-, Bandscheibenschäden, -degenerationen-, oder -erkrankungen, oder für rheumatoide Arthritis eingesetzt werden.

15. Pharmazeutische Zusammensetzung, die eine Kombination des Antikörpers W8B2 oder funktioneller Fragmente des Antikörpers W8B2, der von der bei der Deutschen Sammlung von Mikroorganismen und Zellkulturen hinterlegten Hybridomzelllinie mit der Nr. ACC 2567 produziert wird, und des Anti-körpers 39D5 oder funktioneller Fragmente des Antikörpers 39D5 aufweist, der von der bei der Deutschen Sammlung von Mikroorganismen und Zellkulturen hinterlegten Hybridomzelllinie Nr. ACC 2930 produziert wird.

16. Pharmazeutische Zusammensetzung, enthaltend TNAP⁺ CD56⁺ -Stammzellen, die gemäß dem Verfahren nach einem der Ansprüche 6 und 7 oder 8 oder 9 oder 10 isoliert und/oder identifiziert wurden, sowie zumindest einen pharmazeutisch akzeptierbaren Träger, und ggf. therapeutisch wirksame Substanzen.

17. Kit, enthaltend eine Kombination des Antikörpers W8B2 oder funktioneller Fragmente des Antikörpers W8B2, der von der bei der Deutschen Sammlung von Mikroorganismen und Zellkulturen hinterlegten Hybridomzelllinie mit der Nr. ACC 2567 produziert wird, und des Antikörpers 39D5 oder funktionelle Fragmente des Antikörpers 39D5, der von der bei der Deutschen Sammlung von Mikroorganismen und Zellkulturen hinterlegten Hybridomzelllinie Nr. ACC 2930 produziert wird.

## Claims

1. In vitro use of an antibody or functional fragments of the antibody which binds to the antigen TNAP, in combination with an antibody or functional fragments of the antibody which binds to CD56, for the isolation and/or identification of stem cells with adipocytic, chondrocytic and pancreatic differentiation potential.

2. The use as claimed in claim 1, **characterized in that** the anti-TNAP antibody is selected from the group of:
- the antibody W8B2 which is produced by the cell line deposited at the German Collection of Microorganisms and Cell Cultures with the No. ACC 2567,
- functional fragments of the antibody W8B2 which bind to the antigen TNAP, which antibody is produced by the cell line deposited at the German Collection of Microorganisms and Cell Cultures with the No. ACC 2567, and
- an antibody which binds to the same epitope as the antibody W8B2 which is produced by the cell line deposited at the German Collection of Microorganisms and Cell Cultures with the No. ACC 2567.

3. The use as claimed in claim 2, **characterized in that** the antibody binding to CD56 is selected from the group of:
- the antibody 39D5 which is produced by the cell line deposited at the German Collection of Microorganisms and Cell Cultures with the No. ACC 2930,
- functional fragments of the antibody 39D5 which bind to CD56, which antibody is produced by the cell line deposited at the German Collection of Microorganisms and Cell Cultures with the No. ACC 2930,
- an antibody which binds to the same antigen as the antibody 39D5 which is produced by the cell line deposited at the German Collection of Microorganisms and Cell Cultures with the No. ACC 2930, and
- an antibody which binds to the same epitope as the antibody 39D5 which is produced by the cell line deposited at the German Collection of Microorganisms and Cell Cultures with the No. ACC 2930.

4. The use as claimed in one of claims 1 to 3, **characterized in that** in addition to the combination of an anti-TNAP and an antibody directed against CD56, an antibody directed against CD271, or functional fragments of the antibody binding to CD271 is used.

5. A Method for the isolation and/or identification of mesenchymal stem cells with adipocytic differentiation potential, **characterized in that** it comprises the following steps:
a) contacting a sample which contains mesenchymal stem cells with an antibody or with functional fragments of the antibody which binds to the antigen TNAP,
b) contacting the sample from step a) with an antibody or with functional fragments of the antibody which binds to CD56, and
c) isolating and/or identifying of cells to which the antibody or functional fragments of the antibody which binds to the antigen TNAP, but not the antibody or functional fragments of the antibody which binds to CD56 has bound.

6. A method for the isolation and/or identification of mesenchymal stem cells with chondrocytic differentiation potential, **characterized in that** it comprises the following steps:
a) contacting a sample which contains mesenchymal stem cells with an antibody or with functional fragments of the antibody which binds to the antigen TNAP,
b) contacting the sample from step a) with an antibody or with functional fragments of the antibody which binds to CD56, and
c) isolating and/or identifying of cells to which both the antibody or functional fragments of the antibody which binds to the antigen TNAP and also the antibody or functional fragments of the antibody which binds to CD56 have bound.

7. The method as claimed in claim 6, **characterized in that** it comprises the further step c'):
c') contacting the sample from step b) with an antibody or with functional fragments of the antibody which binds to CD271.

8. The method as claimed in one of claims 5 to 7, **characterized in that** the anti-TNAP antibody used in step a) is selected from the group of:
- the antibody W8B2 which is produced by the hybridoma cell line deposited at the German Collection of Microorganisms and Cell Cultures with the No. ACC 2567,
- functional fragments of the antibody W8B2, which bind to the antigen TNAP, and
- antibodies which bind to the same epitope as the antibody W8B2 which is produced by the hybridoma cell line deposited at the German Collection of Microorganisms and Cell Cultures with the No. ACC 2567.

9. The method as claimed in one of claims 5 to 8, **characterized in that** the antibody binding to CD56 used in step b) is selected from the group of:
- the antibody 39D5 which is produced by the hybridoma cell line deposited at the German Collection of Microorganisms and Cell Cultures with the No. ACC 2930,
- functional fragments of the antibody 39D5 which bind to CD56, and
- antibodies which bind to the same epitope as the antibody 39D5 which is produced by the hybridoma cell line deposited at the German Collection of Microorganisms and Cell Cultures with the No. ACC 2930.

10. The method as claimed in one of claims 5 to 9, **characterized in that** the steps a), b) and c') are performed simultaneously, one after another or in reverse order.

11. TNAP⁺ CD56⁺ stem cells which were isolated and/or identified with a method as claimed in one of claims 6 and 7 or 8 or 9 or 10 for use in therapy or diagnosis.

12. Use of TNAP⁺ CD56⁺ stem cells which were isolated and/or identified with a method as claimed in one of claims 6 and 7 or 8 or 9 or 10 for the defined generation of chondrocytes, adipocytes and pancreas-like cells.

13. The use as claimed in claim 12, **characterized in that** the TNAP⁺ CD56⁺ stem cells which were isolated and/or identified with a method as claimed in one of claims 6 to 10, and were differentiated into chondrocytes, adipocytes and pancreas-like cells, are used for therapy and/or prophylaxis.

14. TNAP⁺ CD56⁺ stem cells which were isolated and/or identified with a method as claimed in one of claims 6 and 7 or 8 or 9 or 10 for use in the therapy and/or prophylaxis of cartilage, bone or disk damage, degeneration or diseases, or for rheumatoid arthritis are used.

15. A pharmaceutical composition which comprises a combination of the antibody W8B2 or functional fragments of the antibody W8B2 which is produced by the hybridoma cell line deposited at the German Collection of Microorganisms and Cell Cultures with the No. ACC 2567, and the antibody 39D5 or functional fragments of the antibody 39D5 which is produced by the hybridoma cell line deposited at the German Collection of Microorganisms and Cell Cultures with the No. ACC 2930.

16. A pharmaceutical composition, containing TNAP⁺ CD56⁺ stem cells which were isolated and/or identified according to the method as claimed in one of claims 6 and 7 or 8 or 9 or 10, and at least one pharmaceutically acceptable carrier, and if necessary therapeutically active substances.

17. A kit, containing comprises a combination of the antibody W8B2 or functional fragments of the antibody W8B2 which is produced by the hybridoma cell line deposited at the German Collection of Microorganisms and Cell Cultures with the No. ACC 2567, and the antibody 39D5 or functional fragments of the antibody 39D5 which is produced by the hybridoma cell line deposited at the German Collection of Microorganisms and Cell Cultures with the No. ACC 2930.

## Revendications

1. Utilisation in vitro d'un anticorps ou de fragments fonctionnels de l'anticorps, qui se lie à l'antigène TNAP, en combinaison avec un anticorps ou des fragments fonctionnels de l'anticorps qui se lie à CD56, pour l'isolement de cellules souches ayant un potentiel de différenciation adipocytaire, chondrocytaire et pancréatique.

2. Utilisation selon la revendication 1, **caractérisée en ce que** l'anticorps anti-TNAP est choisi dans le groupe :
- l'anticorps W8B2, qui est produit par la lignée cellulaire déposée auprès de la Deutsche Sammlung von Mikroorganismen und Zellkulturen sous le numéro ACC 2567,
- les fragments fonctionnels de l'anticorps W8B2, qui se lient à l'antigène TNAP, qui est produit par la lignée cellulaire déposée auprès de la Deutsche Sammlung von Mikroorganismen und Zellkulturen sous le numéro ACC 2567, et
- un anticorps qui se lie au même épitope que l'antigène W8B2, qui est produit par la lignée cellulaire déposée auprès de la Deutsche Sammlung von Mikroorganismen und Zellkulturen sous le numéro ACC 2567.

3. Utilisation selon la revendication 2, **caractérisée en ce que** l'anticorps se liant à CD56 est choisi dans le groupe :
- l'anticorps 39D5, qui est produit par la lignée cellulaire déposée auprès de la Deutsche Sammlung von Mikroorganismen und Zellkulturen sous le numéro ACC 2930,
- les fragments fonctionnels de l'anticorps 39D5, qui se lient à CD56, qui est produit par la lignée cellulaire déposée auprès de la Deutsche Sammlung von Mikroorganismen und Zellkulturen sous le numéro ACC 2930,
- un anticorps qui se lie au même antigène que l'anticorps 39D5, qui est produit par la lignée cellulaire déposée auprès de la Deutsche Sammlung von Mikroorganismen und Zellkulturen sous le numéro ACC 2930, et
- un anticorps qui se lie au même épitope que l'anticorps 39D5, qui est produit par la lignée cellulaire déposée auprès de la Deutsche Sammlung von Mikroorganismen und Zellkulturen sous le numéro ACC 2930.

4. Utilisation selon l'une des revendications 1 à 3, **caractérisée en ce que**, en plus d'une combinaison d'un anticorps anti-TNAP et d'un anticorps dirigé contre CD56, on utilise encore un anticorps dirigé contre CD271, ou des fragments fonctionnels de l'anticorps qui se lient à CD271.

5. Procédé d'isolement et/ou d'identification de cellules souches mésenchymateuses ayant un potentiel de différenciation adipocytaire, **caractérisé en ce qu'**il comprend les étapes suivantes :
a) mise en contact d'un échantillon qui contient des cellules souches mésenchymateuses avec un anticorps ou avec des fragments fonctionnels de l'anticorps qui se lie à l'antigène TNAP,
b) mise en contact de l'échantillon de l'étape a) avec un anticorps qui se lie à CD56, ou avec des fragments fonctionnels de l'anticorps qui se lient à CD56, et
c) isolement et/ou identification de cellules auxquelles l'anticorps ou des fragments fonctionnels de l'anticorps qui se lie à l'antigène TNAP se sont liés, mais auxquels l'anticorps ou des fragments fonctionnels de l'anticorps qui se lie à CD56 ne se sont pas liés.

6. Procédé d'isolement et/ou d'identification de cellules souches mésenchymateuses ayant un potentiel de différenciation chondrocytaire, **caractérisé en ce qu'**il comprend les étapes suivantes :
a) mise en contact d'un échantillon qui contient des cellules souches mésenchymateuses avec un anticorps ou avec des fragments fonctionnels de l'anticorps qui se lie à l'antigène TNAP,
b) mise en contact de l'échantillon de l'étape a) avec un anticorps ou avec des fragments fonctionnels de l'anticorps qui se lie à CD56, et
c) isolement et/ou identification de cellules auxquelles tant l'anticorps ou des fragments fonctionnels de l'anticorps, qui se lie à l'antigène TNAP, que l'anticorps ou des fragments fonctionnels de l'anticorps qui se lie à CD56, se sont liés.

7. Procédé selon la revendication 6, **caractérisé en ce qu'**il comprend une étape supplémentaire c') :
c') mise en contact de l'échantillon de l'étape b) avec un anticorps ou des fragments fonctionnels de l'anticorps qui se lie à CD271.

8. Procédé selon l'une des revendications 5 à 7, **caractérisé en ce que** l'anticorps anti-TNAP utilisé dans l'étape a) est choisi dans le groupe :
- l'anticorps W8B2, qui est produit par la lignée de cellules d'hybridome déposée auprès de la Deutsche Sammlung von Mikroorganismen und Zellkulturen sous le numéro ACC 2567,
- des fragments fonctionnels de l'anticorps W8B2, qui se lient à l'antigène TNAP, et
- des anticorps qui se lient au même épitope que l'anticorps W8B2, qui est produit par la lignée de cellules d'hybridome déposée auprès de la Deutsche Sammlung von Mikroorganismen und Zellkulturen sous le numéro ACC 2567.

9. Procédé selon l'une des revendications 5 à 8, **caractérisé en ce que** l'anticorps utilisé dans l'étape b), se liant à CD56, est choisi dans le groupe :
- l'anticorps 39D5, qui est produit par la lignée de cellules d'hybridome déposée auprès de la Deutsche Sammlung von Mikroorganismen und Zellkulturen sous le numéro ACC 2930,
- des fragments fonctionnels de l'anticorps 39D5, qui se lient à CD56, et
- des anticorps qui se lient au même épitope que l'anticorps 39D5, qui est produit par la lignée de cellules d'hybridome déposée auprès de la Deutsche Sammlung von Mikroorganismen und Zellkulturen sous le numéro ACC 2930.

10. Procédé selon l'une des revendications 5 à 9, **caractérisé en ce que** les étapes a), b) et c') sont réalisées simultanément, successivement ou dans l'ordre inverse.

11. Cellules souches TNAP⁺ CD56⁺, qui ont été isolées et/ou identifiées par un procédé selon l'une des revendications 6 et 7 ou 8 ou 9 ou 10, pour utilisation en thérapie ou diagnostic.

12. Utilisation de cellules souches TNAP⁺ CD56⁺, qui ont été isolées et/ou identifiées par un procédé selon l'une des revendications 6 et 7 ou 8 ou 9 ou 10, pour la production définie de chondrocytes et de cellules analogues à des cellules pancréatiques.

13. Utilisation selon la revendication 12, **caractérisée en ce que** les cellules souches TNAP⁺ CD56⁺ qui ont été isolées et identifiées par un procédé selon l'une des revendications 6 à 10 et ont été différenciées en chondrocytes et cellules analogues à des cellules pancréatiques, peuvent être utilisées en thérapie et/ou prophylaxie.

14. Cellules souches TNAP⁺ CD56⁺ qui ont été isolées et/ou identifiées par un procédé selon l'une des revendications 6 et 7 ou 8 ou 9 ou 10, pour utilisation en thérapie et/ou prophylaxie des détériorations, dégénérescences ou maladies du cartilage, des os, des disques intervertébraux, ou pour la polyarthrite rhumatoïde.

15. Composition pharmaceutique qui comprend une combinaison de l'anticorps W8B2 ou de fragments fonctionnels de l'anticorps W8B2, qui est produit par la lignée de cellules d'hybridome déposée auprès de la Deutsche Sammlung von Mikroorganismen und Zellkulturen sous le numéro ACC 2567, et de l'anticorps 39D5 ou de fragments fonctionnels de l'anticorps 39D5, qui est produit par la lignée de cellules d'hybridome déposée auprès de la Deutsche Sammlung von Mikroorganismen und Zellkulturen sous le numéro ACC 2930.

16. Composition pharmaceutique contenant des cellules souches TNAP⁺ CD56⁺, qui ont été isolées et/ou identifiées par le procédé selon l'une des revendications 6 et 7 ou 8 ou 9 ou 10, ainsi qu'au moins un support pharmaceutiquement acceptable et éventuellement des substances thérapeutiquement actives.

17. Trousse contenant une combinaison de l'anticorps W8B2 ou de fragments fonctionnels de l'anticorps W8B2, qui est produit par la lignée de cellules d'hybridome déposée auprès de la Deutsche Sammlung von Mikroorganismen und Zellkulturen sous le numéro ACC 2567, et de l'anticorps 39D5 ou de fragments fonctionnels de l'anticorps 39D5, qui est produit par la lignée de cellules d'hybridome déposée auprès de la Deutsche Sammlung von Mikroorganismen und Zellkulturen sous le numéro ACC 2930.
